# EUROPEAN PATENT APPLICATION

(11) **EP 4 253 413 A1**
(43) Date of publication of application: **04.10.2023**
(21) Application number: 21914475.5
(22) Date of filing: 29.12.2021
(51) Int. Cl.: C07K 16/24, A61K 39/00, A61P 35/00

(54) **HUMAN LIFR ANTIGEN BINDING PROTEIN, PREPARATION METHOD THEREFOR, AND APPLICATION THEREOF**

(30) Priority: 31.12.2020 CN 202011619032
(71) Applicant: Nona Biosciences (Suzhou) Co., Ltd., Suzhou, Jiangsu 215000 (CN); Yinuoke Biomedical Co., Ltd., Nanchang, Jiangxi 330096 (CN)
(72) Inventor: CONG, Yanni, Suzhou, Jiangsu 215123 (CN); ZHANG, Lingbing, Cayman Islands (KY); DENG, Changjing, Suzhou, Jiangsu 215123 (CN); ZHU, Guangbei, Suzhou, Jiangsu 215123 (CN); ZHOU, Liang, Suzhou, Jiangsu 215123 (CN); MA, Ruipeng, Suzhou, Jiangsu 215123 (CN); CHEN, Yin, Suzhou, Jiangsu 215123 (CN); LIU, Lile, Suzhou, Jiangsu 215123 (CN)
(74) Representative: Maiwald GmbH
(86) International application number: PCT/CN2021/142404
(87) International publication number: WO 2022/143743

(57) **Abstract**

Provided are a human LIFR antigen binding protein, a preparation method therefor, and an application thereof. A series of human LIFR antibodies having biological functions are obtained using human LIFR-ECD protein immune H2L2 humanized antibody transgenic mice by means of single B cell cloning technology (SBC), single B cell sequencing analysis, and Fc recombination. The method greatly improves the discovery efficiency and the yield of antibody drugs, and the obtained human LIFR antibody has good affinity and can block the binding of huLIF proteins to huLIFR/gp130 cells, thereby inhibiting signaling pathways such as STAT3, further inhibiting the growth of tumors, and achieving the purpose of tumor prevention and treatment.

## Description

### TECHNICAL FIELD

The present invention relates to the field of biomedicine, and specifically relates to a human LIFR antigen binding protein and its preparation method and application.

### BACKGROUND

Leukemia inhibitory factor (LIF) is a cytokine with multiple biological functions, named for its ability to induce differentiation of M1 myeloid leukemia cells to normal cells. LIF belongs to the interleukin-6 (IL-6) family, which also includes IL-11, IL-27, ciliary neurotrophic factor (CNTF), cardiotrophin-1 (CT-1) and oncostatin M (OSM), etc., these cytokines share gp130 receptor with LIF. Therefore, LIF has a unique biological role that overlaps with other family members.

The LIF protein in mammals consists of 180 amino acid residues and is highly conserved. The human LIF gene is on chromosome 22q12, while the mouse LIF gene is located on chromosome 11, The mouse and human LIF genes are homologous> 75%. Mature LIF protein is a highly glycosylated secretory protein that can be secreted by a variety of adult cells such as oocytes and human embryo extraembryonic cells, endometrial cells, fibroblasts, hepatocytes, and mononuclear macrophages. The glycosylation of LIF protein from different tissues is different. The most studied one is a secreted and variable glycosylated protein (34-63 kDa), which has induced autocrine and extensive paracrine effects. This effect may be contradictory due to different cell types and backgrounds, such as proliferation and differentiation, survival and apoptosis.

LIF mainly binds to its specific LIF receptor (LIF receptor, LIFR), and then recruits gp 130 receptor subunits to form a high-affinity heterodimeric receptor complex to induce the activation of downstream JAK/STAT3, PI3K/AKT, ERK1/2, MAPK, and other signal pathways to exert various cell functions and regulate cell proliferation and survival. And LIFR is abundantly expressed in various tissues and organs such as the central nervous system, kidney, liver, bone, and uterus. The wide distribution of LIF and LIFR and several LIF signal pathways determine the complexity and diversity of LIF functions.

The LIF pathway is a promising new target in the field of immunology. Studies have reported that the LIF signaling pathway plays an important role in the occurrence and progression of tumors, enhance tumor cell migration and infiltration, and promote tumor metastasis; at the same time, it can regulate a variety of immune cells in the tumor microenvironment, including effector T cells (T -eff), regulatory T cells (T-reg), Th17 and myeloid cells, etc.Studies have also shown that the LIF signaling pathway can promote the activity of tumor-initiating cells (CIC) and enhance resistance to anti-tumor treatments (chemotherapy and radiotherapy). In many cancers, high expression of LIF is associated with poor prognosis.

Patent CN111378036A discloses a preparation method of anti-human leukemia inhibitory factor (LIF) monoclonal antibody and its application, which adopts mouse hybridoma cell technology to prepare anti-human leukemia inhibitory factor monoclonal antibody, but the efficiency of antibody screening and product yield by this method is low. Patent CN111868086A discloses a series of anti-LIF antibodies and their applications. The LIF antibody can bind to the unique epitope of LIF, thereby inhibiting the biological activity of LIF.

Currently, clinically available antibodies against LIF targets include Northern biologics' MSC-1 antibody, this antibody completed the phase I clinical study in 2019. Currently, several clinical efficacy trials are being planned for the use of MSC-1 in the treatment of a range of solid tumors, including pancreatic cancer and lung cancer.MSC-1 antibody is a humanized monoclonal antibody (IgG1) that binds to the immunosuppressive human cytokine LIF for the treatment of adult patients with advanced solid tumors.

However, the MSC-1 antibody can only bind to the specific epitope of LIF, and therefore cannot completely compete for the binding of LIF to the receptor. EC359 is a small molecule inhibitor targeting LIFR < EC359:A First-in-Class Small-Molecule Inhibitor for Targeting Oncogenic LIFR Signaling in Triple-Negative Breast Cancer, DOI:10.1158/1535-7163.MCT-18-1258) .

Therapeutic antibodies against LIFR have not been reported in the prior art; therefore, there is an urgent need to develop high-efficiency and specific blocking antigen binding proteins against the LIF receptor (LIFR) for the treatment of LIFR-related diseases.

### SUMMARY

In view of the above-mentioned shortcomings, the present invention provides for the first time a fully human LIFR antigen binding protein and its preparation method and application. The present invention uses human LIFR-ECD protein to immunize H2L2 human antibody transgenic mice, and uses single B cell cloning technology (SBC) to obtain a series of human LIFR antibodies with biological functions. This method greatly improves the efficiency and yield of antibody drug discovery. The human LIFR antibody obtained has good affinity and can block the binding of huLIF protein to huLIFR/gp130 cells, thereby inhibiting signal pathways such as STAT3, suppressing tumor growth, and achieving the purpose of tumor prevention and treatment.

To achieve the above-mentioned purpose of the invention, the technical solution of the invention is as follows:
In one aspect, the present invention provides a human LIFR antigen binding protein.

Specifically, the human LIFR antigen binding protein comprises the following complementarity determining regions:
(1) the heavy chain complementarity determining region 1 HCDR1 comprising the amino acid sequence set forth in any one of SEQ ID NO: 1 to SEQ ID NO: 4 or a variant sequence thereof;
(2) the heavy chain complementarity determining region 2 HCDR2 comprising the amino acid sequence set forth in any one of SEQ ID NO: 5 to SEQ ID NO: 15 or a variant sequence thereof;
(3) the heavy chain complementarity determining region 3 HCDR3 comprising the amino acid sequence set forth in any one of SEQ ID NO: 16-SEQ ID NO: 25 or a variant sequence thereof;
(4) the light chain complementarity determining region 1 LCDR1 comprising the amino acid sequence set forth in any one of SEQ ID NO: 26-SEQ ID NO: 32 or a variant sequence thereof
(5) the light chain complementarity determining region 2 LCDR2 comprising the amino acid sequence set forth in any one of SEQ ID NO: 33-SEQ ID NO: 40 or a variant sequence thereof;
(6) the light chain complementarity determining region 3 LCDR3 comprising the amino acid sequence set forth in any one of SEQ ID NO: 41-SEQ ID NO: 45 or a variant sequence thereof;
preferably, the variant sequence is a CDR sequence having one or several amino acid substitutions, deletions or additions compared to its souce CDR; the substitutions are conservative substitutions.

More specifically, the human LIFR antigen binding protein comprises the following complementarity determining regions:
(1) the heavy chain complementarity determining region 1 HCDR1 comprising the amino acid sequence set forth in GFTFSSYGMX₁ or the amino acid sequence set forth in SEQ ID NO: 4: GFTFSNYAMT, wherein X₁ = H, D or N; and/or
(2) the heavy chain complementarity determining region 2 HCDR2 comprising the amino acid sequence set forth in VIWYDGX₂NKYYX₃DSVKG, the amino acid sequence set forth in VIWFDGSX₄KYYADSVKG, the amino acid sequence set forth in SEQ ID NO: 7: VIWYDGSNKFYADSVRG, the amino acid sequence set forth in SEQ ID NO: 14: TISGSGAFTYYADAVKG, the amino acid sequence set forth in SEQ ID NO: 15: VISGSGFLTYYADAVKG, wherein X₂=N or S, X₃=E, A or T, X₄=N, I, L or V; and/or
(3) the heavy chain complementarity determining region 3 HCDR3 comprising the amino acid sequence set forth in SEQ ID NO: 16: DGESSMVRGLLNWFDP, the amino acid sequence set forth in SEQ ID NO: 17: ELRYFDWLLSPFDY, the amino acid sequence set forth in SEQ ID NO: 21: GERTLDL, the amino acid sequence set forth in ELWFGELLSPX₅DF, the amino acid sequence set forth in GQLVX₆DX₇, or the amino acid sequence set forth in GGILTGFDXs, wherein X₅=L or F, X₆=G or Q, X₇=Y, F or L, X₈=N or Y; and/or
(4) the light chain complementarity determining region 1 LCDRlcomprising the amino acid sequence set forth in RASQSX₉SSSX₁₀LA, the amino acid sequence set forth in RASQSISSX₁₁LX₁₂, or the amino acid sequence set forth in RASQNLNSNLA, wherein X₉=V or I, X₁₀=Y or F, X₁₁=Y, W or N, X₁₂=N or A; and/or
(5) the light chain complementarity determining region 2 LCDR2 comprising the amino acid sequence set forth in GX₁₃SSRAT, the amino acid sequence set forth in KASX₁₄LEX₁₅, the amino acid sequence set forth in SEQ ID NO: 35: AASNRAT, the amino acid sequence set forth in SEQ ID NO: 36: AASSLQS, the amino acid sequence set forth in SEQ ID NO: 40: GASTRAP,wherein X₁₃=A or T, X₁₄=S Or N, X₁₅=S or N; and/or
(6) the light chain complementarity determining region 3 LCDR3 comprising the amino acid sequence set forth in SEQ ID NO: 41: QQYGSSPFT, the amino acid sequence set forth in SEQ ID NO: 42: QQSYSTPLT, the amino acid sequence set forth in SEQ ID NO: 43: QQYKSFSPGGLT, the amino acid sequence set forth in SEQ ID NO: 44: QQYKSNPLT, the amino acid sequence set forth in SEQ ID NO: 45: QQYNNWPRT.

Further specifically, the human LIFR antigen binding protein comprises:
(1) the heavy chain variable region VH comprising the amino acid sequence set forh in any one of SEQ ID NO:46-SEQ ID NO:59; and/or the light chain variable region VL comprising the amino acid sequence set forth any one of SEQ ID NO:60-SEQ ID NO:69; or
(2) VH has at least 70%, at least 80%, at least 85%, at least 90%, at least 91%, at least 92%, at least 93%, at least 94%, at least 95%, at least 96%, at least 97%, at least 98%, or at least 99% sequence identity to any one of VH in (1); and/or, VLhas at least 70%, at least 80%, at least 85%, at least 90%, at least 91%, at least 92%, at least 93%, at least 94%, at least 95%, at least 96%, at least 97%, at least 98%, or at least 99% sequence identity to any one of VL in (1); or
(3) VH has one or more amino acid substitutions, deletions or additions or any combination thereof compared to any one of VH in (1); and/or, VL has one or more amino acid substitutions, deletions or additions or any combination thereof compared to any one of VL in (1); the substitutions are conservative substitutions.

Futher specifically, the human LIFR antigen binding protein comprises:
(1) aheavy chain HC comprising the amino acid sequence set forth in any one of SEQ ID NO: 70-SEQ ID NO: 83; and/or a light chain LC comprising the amino acid sequence set forth in any one of SEQ ID NO: 84-SEQ ID NO: 93; Or
(2) heavy chain and light chain, wherein the heavy chain has at least 80%, at least 85%, at least 90%, at least 91%, at least 92% , at least 93%, at least 94%, at least 95%, at least 96%, at least 97%, at least 98%, or at least 99% sequence identity to heavy chain in(1); and/or, the light chain has at least 70%, at least 80%, At least 85%, at least 90%, at least 91%, at least 92%, at least 93%, at least 94%, at least 95%, at least 96%, at least 97%, at least 98%, or at least 99% sequence identity to light chain in(1).

Futher specifically, the human LIFR antigen binding protein comprises:
(1) HCDR1 as shown in SEQ ID NO:1, HCDR2 as shown in SEQ ID NO:5, HCDR3 as shown in SEQ ID NO:16, and/or LCDR1 as shown in SEQ ID NO:26, LCDR2 as shown in SEQ ID NO: 33, LCDR3 as shown in SEQ ID NO: 41;
(2) HCDR1 as shown in SEQ ID NO: 1, HCDR2 as shown in SEQ ID NO: 6, HCDR3 as shown in SEQ ID NO: 17, and/or LCDR1 as shown in SEQ ID NO: 27, LCDR2 as shown in SEQ ID NO: 34, LCDR3 as shown in SEQ ID NO: 41;
(3) HCDR1 as shown in SEQ ID NO: 2, HCDR2 as shown in SEQ ID NO: 7, HCDR3 as shown in SEQ ID NO: 18, and/or LCDR1 as shown in SEQ ID NO: 26, LCDR2 as shown in SEQ ID NO: 33, LCDR3 as shown in SEQ ID NO: 41;
(4) HCDR1 as shown in SEQ ID NO: 2, HCDR2 as shown in SEQ ID NO: 7, HCDR3 as shown in SEQ ID NO: 19, and/or LCDR1 as shown in SEQ ID NO: 26, LCDR2 as shown in SEQ ID NO: 33, LCDR3 as shown in SEQ ID NO: 41;
(5) HCDR1 as shown in SEQ ID NO: 2, HCDR2 as shown in SEQ ID NO: 8, HCDR3 as shown in SEQ ID NO: 19, and/or LCDR1 as shown in SEQ ID NO: 28, LCDR2 as shown in SEQ ID NO: 35, LCDR3 as shown in SEQ ID NO: 41;
(6) HCDR1 as shown in SEQ ID NO: 2, HCDR2 as shown in SEQ ID NO: 9, HCDR3 as shown in SEQ ID NO: 19, and/or LCDR1 as shown in SEQ ID NO: 26, LCDR2 as shown in SEQ ID NO: 35, LCDR3 as shown in SEQ ID NO: 41;
(7) HCDR1 as shown in SEQ ID NO: 1, HCDR2 as shown in SEQ ID NO: 10, HCDR3 as shown in SEQ ID NO: 20, and/or LCDR1 as shown in SEQ ID NO: 29, LCDR2 as shown in SEQ ID NO: 36, LCDR3 as shown in SEQ ID NO: 42;
(8) HCDR1 as shown in SEQ ID NO: 3, HCDR2 as shown in SEQ ID NO: 10, HCDR3 as shown in SEQ ID NO: 21, and/or LCDR1 as shown in SEQ ID NO: 30, LCDR2 as shown in SEQ ID NO: 37, LCDR3 as shown in SEQ ID NO: 43;
(9) HCDR1 as shown in SEQ ID NO: 3, HCDR2 as shown in SEQ ID NO: 11, HCDR3 as shown in SEQ ID NO: 22, and/or LCDR1 as shown in SEQ ID NO: 30, LCDR2 as shown in SEQ ID NO: 38, LCDR3 as shown in SEQ ID NO: 44;
(10) HCDR1 as shown in SEQ ID NO: 3, HCDR2 as shown in SEQ ID NO: 11, HCDR3 as shown in SEQ ID NO: 22, and/or LCDR1 as shown in SEQ ID NO: 30, LCDR2 as shown in SEQ ID NO: 39, LCDR3 as shown in SEQ ID NO: 44;
(11) HCDR1 as shown in SEQ ID NO: 1, HCDR2 as shown in SEQ ID NO: 12, HCDR3 as shown in SEQ ID NO: 23, and/or LCDR1 as shown in SEQ ID NO: 30, LCDR2 as shown in SEQ ID NO: 38, LCDR3 as shown in SEQ ID NO: 44;
(12) HCDR1 as shown in SEQ ID NO: 1, HCDR2 as shown in SEQ ID NO: 11, HCDR3 as shown in SEQ ID NO: 22, and/or LCDR1 as shown in SEQ ID NO: 30, LCDR2 as shown in SEQ ID NO: 38, LCDR3 as shown in SEQ ID NO: 44;
(13) HCDR1 as shown in SEQ ID NO: 1, HCDR2 as shown in SEQ ID NO: 13, HCDR3 as shown in SEQ ID NO: 22, and/or LCDR1 as shown in SEQ ID NO: 30, LCDR2 as shown in SEQ ID NO: 38, LCDR3 as shown in SEQ ID NO: 44;
(14) HCDR1 as shown in SEQ ID NO: 4, HCDR2 as shown in SEQ ID NO: 14, HCDR3 as shown in SEQ ID NO: 24, and/or LCDR1 as shown in SEQ ID NO: 31, LCDR2 as shown in SEQ ID NO: 37, LCDR3 as shown in SEQ ID NO: 43;
(15) HCDR1 as shown in SEQ ID NO: 4, HCDR2 as shown in SEQ ID NO: 15, HCDR3 as shown in SEQ ID NO: 25, and/or LCDR1 as shown in SEQ ID NO: 32, LCDR2 as shown in SEQ ID NO:40, and the LCDR3 as shown in SEQ ID NO:45.

Preferably, The human LIFR antigen-binding protein was subjected to sequence similarity analysis using Mega software, and included the following five groups:
Group 1: the antigen-binding protein specified in (1);
Group 2: the antigen-binding protein specified in (2), (3) and (4);
Group 3: the antigen-binding protein specified in (5) and (6);
Group 4: the antigen-binding protein specified in (7), (8), (9), (10), (11), (12), (13);
Group 5: the antigen-binding protein specified in (14) and (15).

Futher specifically, the human LIFR antigen binding protein comprises:
(1) VH as shown in SEQ ID NO: 46, and/or VL as shown in SEQ ID NO: 60;
(2) VH as shown in SEQ ID NO: 47, and/or VL as shown in SEQ ID NO: 61;
(3) VH as shown in SEQ ID NO: 48, and/or VL as shown in SEQ ID NO: 62;
(4) VH as shown in SEQ ID NO: 49, and/or VL as shown in SEQ ID NO: 62;
(5) VH as shown in SEQ ID NO: 50, and/or VL as shown in SEQ ID NO: 63;
(6) VH as shown in SEQ ID NO: 51, and/or VL as shown in SEQ ID NO: 64;
(7) VH as shown in SEQ ID NO: 52, and/or VL as shown in SEQ ID NO: 65;
(8) VH as shown in SEQ ID NO:53, and/or VL as shown in SEQ ID NO:66;
(9) VH as shown in SEQ ID NO: 54 and/or VL as shown in SEQ ID NO: 67;
(10) VH as shown in SEQ ID NO: 54 and/or VL as shown in SEQ ID NO: 68;
(11) VH as shown in SEQ ID NO: 55, and/or VL as shown in SEQ ID NO: 67;
(12) VH as shown in SEQ ID NO: 56, and/or VL as shown in SEQ ID NO: 67;
(13) VH as shown in SEQ ID NO: 57, and/or VL as shown in SEQ ID NO: 67;
(14) VH as shown in SEQ ID NO: 58, and/or VL as shown in SEQ ID NO: 66;
(15) VH as shown in SEQ ID NO: 59, and/or VL as shown in SEQ ID NO: 69;

or,VH has at least 70%, at least 80%, at least 85%, at least 90%, at least 91%, at least 92%, at least 93%, at least 94%, at least 95%, at least 96%, at least 97%, at least 98%, or at least 99% sequence identity to any one of VH in (1)-(15); and/or, VLhas at least 70%, at least 80%, at least 85%, at least 90%, at least 91%, at least 92%, at least 93%, at least 94%, at least 95%, at least 96%, at least 97%, at least 98%, or at least 99% sequence identity to any one of VL in (1)-(15);
or, VH has one or more amino acid substitutions, deletions or additions or any combination thereof compared to any one of VH in (1)-(15); and/or, VLhas one or more amino acid substitutions, deletions or additions or any combination thereof compared to any one of VL in (1)-(15); the substitutions are conservative substitutions.

Futher specifically, the human LIFR antigen binding protein comprises:
(1) HC as shown in SEQ ID NO: 70, and/or LC as shown in SEQ ID NO: 84;
(2) HC as shown in SEQ ID NO: 71, and/or LC as shown in SEQ ID NO: 85;
(3) HC as shown in SEQ ID NO: 72, and/or LC as shown in SEQ ID NO: 86;
(4) HC as shown in SEQ ID NO: 73, and/or LC as shown in SEQ ID NO: 86;
(5) HC as shown in SEQ ID NO: 74, and/or LC as shown in SEQ ID NO: 87;
(6) HC as shown in SEQ ID NO: 75, and/or LC as shown in SEQ ID NO: 88;
(7) HC as shown in SEQ ID NO: 76, and/or LC as shown in SEQ ID NO: 89;
(8) HC as shown in SEQ ID NO: 77, and/or LC as shown in SEQ ID NO: 90;
(9) HC as shown in SEQ ID NO: 78, and/or LC as shown in SEQ ID NO: 91;
(10) HC as shown in SEQ ID NO: 78, and/or LC as shown in SEQ ID NO: 92;
(11) HC as shown in SEQ ID NO: 79, and/or LC as shown in SEQ ID NO: 91;
(12) HC as shown in SEQ ID NO: 80, and/or LC as shown in SEQ ID NO: 91;
(13) HC as shown in SEQ ID NO: 81, and/or LC as shown in SEQ ID NO: 91;
(14) HC as shown in SEQ ID NO: 82, and/or LC as shown in SEQ ID NO: 90;
(15) HC as shown in SEQ ID NO: 83, and/or LC as shown in SEQ ID NO: 93.;
or, the heavy chain has at least 70%, at least 80%, at least 85%, at least 90%, at least 91%, at least 92%, at least 93%, at least 94%, at least 95%, at least 96%, at least 97%, at least 98%, or at least 99% sequence identity to any one of heavy chain in (1)-(15); and/or, the light chain has at least 70%, at least 80%, at least 85%, at least 90%, at least 91%, at least 92%, at least 93%, at least 94%, at least 95%, at least 96%, at least 97%, at least 98%, or at least 99% sequence identity to any one of light chain in (1)-(15).

Futher specifically, the human LIFR antigen binding protein comprises a chimeric antibody, a humanized antibody, or a fully human antibody.

Futher specifically, the above-mentioned human LIFR antigen binding proteins comprises full-length antibodies, Fab, Fab', F(ab')2, Fv, scFv, di-scFv, a bispecific antibody, a multispecific antibody, a heavy chain antibody and/ or a single domain antibody, or a monoclonal antibody and/or a polyclonal antibody prepared from the above antibodies.

In another aspect, the present invention provides a series of nucleic acid molecules encoding the human LIFR antigen binding protein.

Specifically, the nucleic acid molecule comprises one or more codon-optimized nucleic acid molecules.

In another aspect, the present invention provides a series of vectors, the vectors comprising one or more nucleic acid molecules as described in this application.

Specifically, the vectors include, but are not limited to, plasmids, viruses, and bacteriophages.

In another aspect, the present invention provides a series of host cells comprise the above nucleic acid molecule or the above vector.

Specifically, the host cells include, but are not limited to, microorganisms, plants, or animal cells. The vectors of the present invention can be introduced into the host cells by methods known to those skilled in the art, such as electroporation, lipofectine transfection., lipofectamin transfection and other methods.

In another aspect, the present invention provides a chimeric antigen receptor, the chimeric antigen receptor comprising the above-mentioned human LIFR antigen binding protein.

In another aspect, the present invention provides an immune cell, the immune cell comprising the above-mentioned chimeric antigen receptor.

In another aspect, the present invention provides an antigen binding protein derivative, which comprises the above-mentioned human LIFR antigen binding protein and a detectable label molecule.

Specifically, the detectable labeling molecule is an enzyme (such as horseradish peroxidase), a radionuclide, a fluorescent dye, a luminescent substance (such as a chemiluminescent substance), or biotin.

In another aspect, the present invention provides a multispecific antibody, which comprises the above-mentioned human LIFR antigen binding protein, and another antibody or fragment thereof or antibody analogue.

Specifically, the multispecific antibody is a bispecific antibody or a trispecific antibody or a tetraspecific antibody.

In yet another aspect, the present invention provides an antibody-drug conjugate. The antibody-drug conjugate comprises an antibody portion and a conjugated moiety. The antibody portion includes the above-mentioned human LIFR antigen-binding protein, and the conjugated moiety includes, but not limited to a detectable label, a drug, a toxin, a cytokine, a radionuclide, an enzyme, or a combination thereof, the antibody portion and the conjugated moiety are coupled through a chemical bond or a linker.

In another aspect, the present invention provides a pharmaceutical composition, and the composition comprises the above-mentioned human LIFR antigen binding protein, nucleic acid molecule, vector, host cell, chimeric antigen receptor, immune cell, antigen binding protein derivatives, multispecific antibodies and/or antibody-drug conjugates.

Specifically, the pharmaceutical composition further comprises an optional pharmaceutically acceptable carrier.

Futher specifically, the pharmaceutically acceptable carrier includes, but is not limited to a diluent, an excipient, a filler, a wetting agent, a disintegrant, a flavoring agent and a binder.

Specifically, the pharmaceutical composition also comprises a therapeutic agent for combinational use, which includes, but is not limited to, a chemotherapeutic agent, a radiotherapy agent, an immunosuppressive agent, and a cytotoxic drug.

In another aspect, the present invention provides a method for producing the above-mentioned human LIFR antigen binding protein, and the method comprises culturing the above-mentioned host cell under the condition that the antigen binding protein is expressed.

Specifically, the method includes the following steps:
(1) using LIFR-ECD protein as antigen to immunize mice;
(2) screening of antigen-specific B cells that secrete antibodies;
(3) restoring the heavy chain and light chain sequences of the antibody from the single B cell selected in step (2);
(4) the antibody heavy chain and light chain recovered in step (3) were recombined with human Fc and introduced into host cells, the human LIFR antigen binding protein is prepared by cell culture and purification.

Futher specifically, the mouse as described in step (1) is a humanized mouse.

Preferably, the humanized mouse is a Harbour H2L2 mouse, and the Harbour H2L2 transgenic mouse strain can produce traditional double-heavy-chain and double-light-chain immunoglobulin antibodies with fully human variable regions.

Futher specifically, the method for screening antibody-secreting antigen-specific B cells described in step (2) is performed using the plasma cell discovery workflow of the Beacon photoelectric system for screening.

Futher specifically, the method for restoring the heavy chain and light chain sequences of the antibody described in step (3) is single B cell sequencing.

Futher specifically, the steps of single B cell sequencing are purifying RNA from single B cell lysate, reversing transcription synthesis of cDNA, amplifying and purifying of cDNA, amplifying of heavy and light chains, cloning and transfecting, Sanger sequencing, making the uniqueness and cluster analysis of the obtained sequence, finally, the paired heavy and light chain DNA sequences were used for plasmid synthesis.In another aspect, the present invention provides the use of the human LIFR antigen binding protein, nucleic acid molecule, vector, host cell, chimeric antigen receptor, immune cell, antigen binding protein derivative, multispecific antibody, antibody-drug conjugate and/or pharmaceutical composition in the preparation of a LIF and/or LIFR blocking drug, a kit and/or a medical device.

Specifically, the LIFR blocking drug, kit and/or device are mainly used in diseases with increased expression of LIF and/or LIFR.

In another aspect, the present invention provides the use of the human LIFR antigen binding protein, nucleic acid molecule, vector, host cell, chimeric antigen receptor, immune cell, antigen binding protein derivative, multispecific antibody, antibody-drug conjugate and/or pharmaceutical composition in the preparation of a drug, a kit and/or a drug delivery device for preventing and/or treating a LIFR-positive disease.

In another aspect, the present invention provides the use of the human LIFR antigen binding protein, antigen binding protein derivatives in the preparation of a LIFR detection reagent or kit.

In another aspect, the present invention provides a LIFR detection method, which uses the above-mentioned human LIFR antigen binding protein to detect LIFR qualitatively or quantitatively, preferably, the detection method is used for non-disease diagnostic or therapeutic purposes.

In yet another aspect, the present invention provides a treatment method for LIFR-positive related diseases, the treatment method is to administer an effective amount of the above-mentioned human LIFR antigen binding protein, immune cells, antigen binding protein derivatives, multi- specific antibodies, antibody-drug conjugates and/or pharmaceutical compositions to a subject in need.

Specifically, the LIFR-positive disease is a tumor, the tumor includes but not limited to cholangiocarcinoma, colorectal cancer, glioma, prostate cancer, ovarian cancer, gastric cancer, nasopharyngeal cancer, breast cancer, bladder cancer, pancreas cancer, non-small cell lung cancer.

In another aspect, the present invention provides a kit comprising the human LIFR antigen binding protein, nucleic acid molecule, vector, host cell, chimeric antigen receptor, immune cell, antigen binding protein derivative, multispecific antibodies, antibody-drug conjugates and/or pharmaceutical compositions, and optionally, instructions.

In another aspect, the present invention provides an administration device comprising: (1) an infusion module for administering the pharmaceutical composition to a subject in need, and (2) optional a drug efficacy monitoring module.

In yet another aspect, the present invention provides the use of LIFR antigen binding protein in the preparation of therapeutic agents for cancer, the cancer is selected from bile duct cancer, colorectal cancer, glioma, prostate cancer, ovarian cancer, gastric cancer, nasopharyngeal cancer, breast cancer, bladder cancer, pancreatic cancer, and non-small cell lung cancer.

Specifically, the LIFR antigen binding protein comprises any one of the above LIFR antigen binding proteins.

Compared with the prior art, the present invention has the following positive and beneficial effects:
(1) The present invention provides for the first time a human LIFR antibody, which is prepared by immunizing humanized antibody transgenic mice (such as Harbour H2L2 mice), and the obtained antibody has fully human antibody amino acid and gene sequences, thereby ensure the lowest possible immunogenicity when used in the human body and removes toxic side effects.
(2) The present invention adopts single B cell cloning technology (SBC) to screen positive clones, which can greatly improve the efficiency and yield of antibody discovery compared to traditional monoclonal antibody screening technology.
(3) The human LIFR antibody prepared by the present invention has high specificity, high affinity, and good anti-tumor activity.
(4) The antibodies of the present invention are fully human antibodies, and thus can be safely administered to human subjects without triggering an immunogenic reaction, which is of great clinical value.

### BRIEF DESCRIPTION OF THE DRAWINGS

Fig.1. Detection diagram of the binding ability of the human LIFR antibody of the present invention to LIFR-ECD at the protein level, wherein A is the detection result of the binding ability of the human LIFR antibody of the present invention to human LIFR-ECD, B is the detection result of the binding ability of the human LIFR antibody of the present invention to monkey LIFR-ECD, C is the detection result of the binding ability of the human LIFR antibody of the present invention to the mouse LIFR-ECD.
Fig.2. Detection diagram of the binding ability of the antibody of the present invention to LIFR and LIFR/gp130 at the cellular level, wherein A is the detection result of the binding ability of the antibody of the present invention to LIFR, B is the detection result of the binding of the antibody of the present invention to LIFR/gp130.
Fig.3. Detection diagram of the ability of the antibody of the present invention to block the binding of LIF to LIFR/gp130.
Fig.4. Detection diagram of the inhibitory effect of the antibody of the present invention on the STAT3 signaling pathway.
Fig.5. PK detection diagram of the antibody of the present invention Balb/c mice.
Fig.6. PK detection diagram of the antibody of the present invention in Sprague Dawley rats, wherein A is the PK detection diagram of the antibody PR300516 in rats, B is the PK detection diagram of the antibody PR301168 in rats, C is the PK average detection diagram of the antibody PR300516 and PR301168 in rats.
Fig.7. PK detection diagram of the antibody of the present invention in monkeys, wherein A is the detection result diagram of a single dose of 10mg/mL, and B is the detection result diagram of a single dose of 100mg/mL.

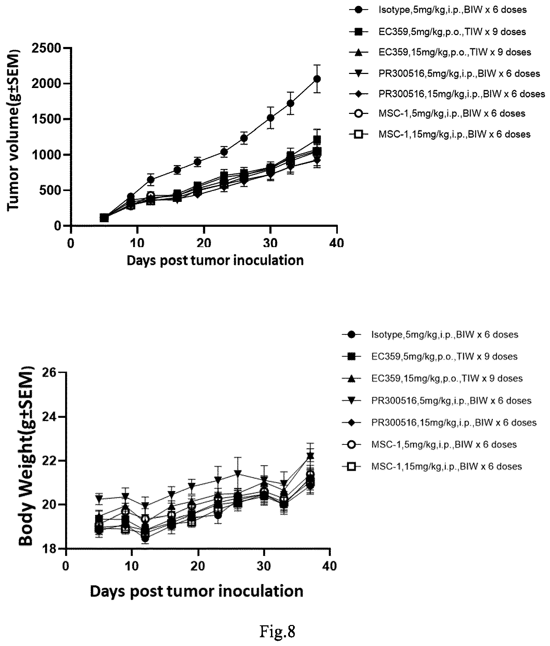

Fig.8. The pharmacodynamic experiment detection diagram of the antibody of the present invention in the Capan-2 mouse model.
Fig.9. The pharmacodynamic experiment detection diagram of the antibody of the present invention in the Capan-1 mouse model
Fig.10. The pharmacodynamic detection diagram of the antibody of the present invention in the NCI-H292 model mouse model.

### DETAILED DESCRIPTION

The present invention will be further described in detail below in conjunction with specific embodiments. The following embodiments are not used to limit the present invention, but are only used to illustrate the present invention. Unless otherwise stated the experimental methods used in the following embodiments are usually in accordance with conventional conditions if no specific conditions are indicated in the embodiments.The materials, reagents used in the following examples can be obtained from commercial sources unless otherwise specified.

### Definition of Terms

To make it easier to understand the present invention, certain technical and scientific terms are specifically defined below. Unless otherwise clearly defined herein, all other technical and scientific terms used herein have meanings commonly understood by those of ordinary skill in the art to which this disclosure belongs.

The three-letter code and one-letter code of amino acids used in the present invention are as described in J. biool. chem, 243, p3558 (1968, ILJPAC-ILJB Committee).

The "antigen-binding protein" in the present invention generally refers to a protein that includes an antigen-binding part, and optionally a scaffold or framework part that allows the antigen-binding part to adopt a conformation that promotes the binding of the antigen-binding protein to the antigen. It may typically comprise an antibody light chain variable region (VL), an antibody heavy chain variable region (VH), or both. The VH and VL regions can be further divided into hypervariable regions called complementarity determining regions (CDR), which are interspersed in more conserved regions called framework regions (FR or FWR). The variable regions of the heavy and light chains contain binding domains that interact with antigens. Examples of antigen-binding proteins include, but are not limited to, antibodies, antigen-binding fragments (Fab, Fab', Fv fragments, F(ab')2, scFv, di-scFv and/or dAb), immunoconjugates, multispecific antibodies (for example, bispecific antibodies), antibody fragments, antibody derivatives, antibody analogs, chimeric antigen receptors or fusion proteins, etc., as long as they show the desired antigen-binding activity.

The "antibody" in the present invention refers to an immunoglobulin, which is a tetrapeptide chain structure composed of two identical heavy chains and two identical light chains linked by interchain disulfide bonds. Immunoglobulins differ in the composition and order of amino acids in the constant region of the heavy chain, and therefore differ in their antigenicity. Accordingly, immunoglobulins can be divided into five categories, or isotypes of immunoglobulins, namely IgM, IgD, IgG, IgA, IgE, and the corresponding heavy chains are µ chain, δ chain, and γ chain, α chain, ε chain. The same class of Ig can be divided into different subclasses according to the differences in the amino acid composition of its hinge region and the number and location of heavy chain disulfide bonds.For example, IgG can be divided into IgG1, IgG2, IgG3, and IgG4. The light chain is divided into a kappa chain or a lambda chain by the difference of the constant region. Each of the five types of Ig can have a kappa chain or a lambda chain.

The sequence of about 110 amino acids near the N-terminus of the antibody heavy and light chains varies greatly and is the variable region (Fv region); the remaining amino acid sequences near the C-terminus are relatively stable and are the constant region. The variable region includes 3 hypervariable regions (HVR) and 4 framework regions (FR) with relatively conservative sequences. Three hypervariable regions determine the specificity of the antibody, also known as complementarity determining regions (CDR). Each light chain variable region (VL or LCVR) and heavy chain variable region (VH or HCVR) consists of 3 CDR regions and 4 FR regions. The sequence from the amino terminal to the carboxy terminal is: FR1, CDR1, FR2, CDR2, FR3, CDR3, FR4. The 3 CDR regions of the light chain refer to LCDR1, LCDR2, and LCDR3, and the 3 CDR regions of the heavy chain refer to HCDR1, HCDR2, and HCDR3.

The term "complementarity determining region" (CDR) refers to one of the six hypervariable regions within the variable structural domain of an antibody that mainly contribute to antigen binding. Generally, there are three CDRs (HCDR1, HCDR2, HCDR3) in each heavy chain variable region, and three CDRs (LCDR1, LCDR2, LCDR3) in each light chain variable region. CDRs can be determined according to various numbering systems known in the art, such as Kabat, Chothia or, AbM or IMGT numbering systems. In the present invention, "Kabat numbering rules" (see Kabat et al. (1991)) are used to determine the amino acid sequence boundaries of CDRs.

The term "framework region" or "FR" residues refers to those amino acid residues in the variable region of an antibody other than the CDR residues as defined above.

The terms "monoclonal antibody" and "mAb" refer to antibodies obtained from a population of substantially homogeneous antibodies, that is, in addition to possible variant antibodies, the individual antibodies constituting the population are identical and/or binding to the same epitope. Unlike polyclonal antibody preparations which usually contain different antibodies directed against different determinants, each monoclonal antibody of the monoclonal antibody preparation is directed against a single determinant on the antigen. therefore. "Monoclonal" refers to the properties of an antibody obtained from a substantially homogeneous antibody population, and should not be interpreted as requiring any specific method to manufacture the antibody. The monoclonal antibodies of the present invention can be prepared by various techniques known to those skilled in the art, including but not limited to hybridoma methods, recombinant DNA methods, phage display methods, transgenic methods, and the like.

The term "humanized monoclonal antibody" refers to the antibody produced by transplanting murine CDR sequences into the human antibody variable region framework, that is, antibodies produced from different types of human germline antibody framework sequences, which overcome the heterologous response induced by chimeric antibodies carrying large amounts of murine protein components. To avoid the decrease of immunogenicity and the resulting decrease in activity, minimal reverse mutations (or back mutations) can be performed on the human antibody variable region framework sequence to maintain activity. To prepare humanized antibodies, methods known in the art can be used to insert murine CDR regions into human framework sequences (see Winter's U.S. Patent Nos. 5,225,539; U.S. Patent Nos. 5,530,101; 5,585,089; 5,693,762 and 6,180,370 by Queen et al; and Lo, Benny, KC, editor, in Antibody Engineering: Methods and Protocols, volume 248, Humana Press, New Jersey, 2004).

Alternatively, transgenic animals can also be used, which can produce no endogenous immunoglobulin after immunization and can produce a complete human antibody library (see, for example, Jakobovits et al., 1993, Proc. Natl. Acad. Sci. USA 90: 2551; Jakobovits et al., 1993, Nature 362:255-258; Bruggermann et al., 1993, Year in Immunology 7:33; and Duchosal et al., 1992, Nature 355:258; Lonberg et al. (1994) Nature 368(6474):856- 859; WO02/43478). Other methods of antibody humanization include phage display technology (Hoogenboom et al., 1991, J. Mol. Biol. 227: 381; Marks et al., J. Mol. Biol. 1991, 222: 581-597; Vaughan et al., 1996, Nature Biotech 14:309). Among them, examples of "transgenic animals" include, but are not limited to, the H2L2 humanized mice of HARBOUR BIOMED.

The terms "specific binding" and "selective binding" refer to a non-random binding reaction between two molecules, such as the reaction between an antibody and the antigen against which it is directed. The strength or affinity of a specific binding interaction can be expressed by the dissociation equilibrium constant (KD) of the interaction. As used herein, the term "KD" refers to the dissociation equilibrium constant of a specific antibody-antigen interaction, which is used to describe the binding affinity between the antibody and the antigen. The smaller the dissociation equilibrium constant, the tighter the antibody-antigen binding, and the higher the affinity between the antibody and the antigen. Generally, antibodies bind with an affinity (KD) of about less than 10-8M, for example, about less than 10-9M, 10-10M, 10-11M, or less.

The term "identity" usually refers to the percentage of amino acid residues or nucleotides in the query sequence that are identical to those in the second reference polypeptide sequence or part thereof after the sequence is aligned, and gaps (GAPS) are introduced when necessary to maximum percent sequence identity, and any conservative substitutions are not considered as part of the sequence identity. The alignment used to determine the percentage of amino acid/nucleotide sequence identity can be achieved in various ways known in the art, for example, using publicly available computer software, such as BLAST, BLAST-2, ALIGN, NEEDLE or Megalign (DNASTAR).Those skilled in the art can determine the appropriate parameters for measuring the alignment, including any algorithm required to achieve the maximum alignment over the full length of the sequence being compared. The percent identity can be determined over the length of the entire determined polypeptide/polynucleotide sequence, or it can be determined over a shorter length, such as the length of a fragment obtained from a larger determined polypeptide/polynucleotide sequence. In the table, figure, or sequence listing, any fragment length supported by the sequence shown herein can be used as the length of the measurable percent identity. A sequence with "% identity" retains important biological activities of the sequence from which it is compared or from which it is derived, such as antibody binding specificity. Nucleotide sequences with "% identity" or nucleotide sequences differing by no more than 3, 6, 15, 30, or 45 nucleotides are capable of performing functions similar to those of the nucleotide sequences they compare or originate from, such as expressing proteins that all specifically bind the same antigen or molecule.

The term "conservative substitution" refers to an amino acid substitution that does not adversely affect or change the expected properties of the protein/polypeptide comprising the amino acid sequence. For example, conservative substitutions can be introduced by standard techniques known in the art such as site-directed mutagenesis and PCR-mediated mutagenesis. Conservative amino acid substitutions include substitutions of amino acid residues with similar side chains, such as those that are physically or functionally similar to the corresponding amino acid residues (e.g., have similar size, shape, charge, chemical properties, including the ability to form covalent bonds or hydrogen bonds, etc.) Families of amino acid residues with similar side chains have been defined in the art. These families include basic side chains (for example, lysine, arginine, and histidine), acidic side chains (for example, aspartic acid, glutamic acid), uncharged polar side chains (for example, glycine, asparagine, glutamine, serine, threonine, tyrosine, cysteine, tryptophan), non-polar side chains (such as alanine, valine, leucine, isoleucine, proline, phenylalanine, methionine), β-branched side chains (e.g., threonine, valine, isoleucine), and aromatic side chains (e.g., tyrosine, Phenylalanine, tryptophan, histidine) amino acids. Therefore, it is preferred to replace the corresponding amino acid residue with another amino acid residue from the same side chain family. Methods for identifying conservative substitutions of amino acids are well known in the art (see, for example, Brummell et al., Biochem. 32:1180-1187 (1993); Kobayashi et al. Protein Eng. 12(10):879-884 (1999); and Burks et al. Proc. Natl Acad. Set USA 94:412-417 (1997), which is incorporated herein by reference).

The term "vector" refers to a nucleic acid delivery vehicle into which polynucleotides can be inserted. When the vector can express the protein encoded by the inserted polynucleotide, the vector is called an expression vector. The vector can be introduced into the host cell through transformation, transduction, or transfection, so that the genetic material elements it carries can be expressed in the host cell. Vectors are well known to those skilled in the art, including but not limited to: plasmids; phagemids; cosmids; artificial chromosomes, such as yeast artificial chromosomes (YAC), bacterial artificial chromosomes (BAC) or P1 derived artificial chromosomes (PAC); Phages such as lambda phage or M13 phage and animal viruses. Animal viruses that can be used as vectors include, but are not limited to, retrovirus (including lentivirus), adenovirus, adeno-associated virus, herpes virus (such as herpes simplex virus), poxvirus, baculovirus, papilloma virus, Papillary polyomavirus (e.g. SV40).A vector can contain a variety of elements that control expression, including but not limited to promoter sequences, transcription initiation sequences, enhancer sequences, selection elements, and reporter genes. In addition, the vector may also contain an origin of replication site.

The term "host cell" generally refers to an individual cell, cell line, or cell culture that can or already contains a plasmid or vector containing the nucleic acid molecule described in this application, or capable of expressing the antibody or antigen-binding fragment thereof described in this application.The cell may include the progeny of a single host cell. Due to natural, accidental, or deliberate mutations, the progeny cells and the original parent cells may not necessarily be completely identical in morphology or genome, but they can express the antibodies or antigen-binding fragments described in this application. The cells can be obtained by transfecting cells in vitro using the vectors described in this application. The cell may be a prokaryotic cell (such as Escherichia coli), or a eukaryotic cell (such as yeast cells, COS cells, Chinese Hamster Ovary (CHO) cells, HeLa cells, HEK293 cells, COS-1 cells, NS0 cells or Myeloma cells). In some cases, the cell may be a mammalian cell. For example, the mammalian cell may be a CHOK1 cell.

The term "pharmaceutically acceptable carrier" refers to a carrier that is pharmacologically and/or physiologically compatible with the subject and the active ingredient, and is well known in the art (see, for example, Remington's Pharmaceutical Sciences. Edited by Gennaro AR, 19th ed Pennsylvania: Mack Publishing Company, 1995), and includes but not limited to: pH adjusters, surfactants, adjuvants, ionic strength enhancers, diluents, agents for maintaining osmotic pressure, agents for delaying absorption, and preservatives. For example, pH adjusting agents include, but are not limited to, phosphate buffer. Surfactants include but are not limited to cationic, anionic, or nonionic surfactants, such as Tween-80. Ionic strength enhancers include, but are not limited to, sodium chloride. Preservatives include, but are not limited to, various antibacterial and antifungal agents, such as parabens, chlorobutanol, phenol, sorbic acid and the like. Agents for maintaining osmotic pressure include, but are not limited to, sugar, NaCl and the like. Agents that delay absorption include, but are not limited to, monostearate and gelatin. Diluents include, but are not limited to, water, aqueous buffers (such as buffered saline), alcohols and polyols (such as glycerol), etc. Preservatives include, but are not limited to, various antibacterial and antifungal agents, such as thimerosal, 2-phenoxyethanol, paraben, chlorobutanol, phenol, sorbic acid and the like. Stabilizers have the meaning commonly understood by those skilled in the art, which can stabilize the desired activity of the active ingredients in the drug, including but not limited to sodium glutamate, gelatin, SPGA, sugars (such as sorbitol, mannitol, starch, sucrose, Lactose, dextran, or glucose), amino acids (such as glutamic acid, glycine), protein (such as dried whey, albumin or casein) or its degradation products (such as lactalbumin hydrolysate), etc.

The term "pharmaceutical composition" generally refers to a formulation that exists in a form that allows the biological activity of the active ingredient to be effective, and does not contain additional ingredients that have unacceptable toxicity to the subject to which the composition is to be administered. The composition is sterile.

The term "subject" refers to a mammal, such as a primate mammal, such as a non-human primate mammal or a human. In certain embodiments, the subject (such as a human) has cancer (including but not limited to cholangiocarcinoma, colorectal cancer, glioma, prostate cancer, ovarian cancer, gastric cancer, nasopharyngeal cancer, breast cancer, Bladder cancer, pancreatic cancer, non-small cell lung cancer), or at risk of suffering from the above diseases.

The term "effective amount" refers to an amount sufficient to obtain or at least partially obtain the desired effect. For example, an effective amount for preventing a disease (e.g., LIFR-positive related) refers to an amount sufficient to prevent, stop or delay the occurrence of a disease (e.g., LIFR-positive related disease); an effective amount for treating a disease refers to an amount sufficient to cure or at least partially prevent disease and its complications in patients who have already suffered from the disease. Measuring such an effective amount is completely intrinsic to those skilled in the art. For example, the effective amount for the purpose of treatment will depend on the severity of the disease being treated, the patient's own immune system status, the general condition of the patient such as age, weight and gender, how the drug is administered, and other treatments administered at the same time, etc.

The term "chimeric antigen receptor" refers to a chimeric antigen receptor T cell that is coupled to the intracellular part of CD3-δ chain or FcεRIγ to form a chimeric protein in vitro through the antigen binding site of an antibody that recognizes a certain tumor antigen, the patient's T cells are genetically transfected to express chimeric antigen receptors, allowing the patient's T cells to be "recoded" to produce large numbers of tumor-specific CAR-T cells.. By adding recoded chimeric antigen receptor T cells into the patient's body, the chimeric antigen receptor acts like a GPS to specifically track and recognize and direct T cells to kill tumor cells. Most chimeric antigen receptors consist of an extracellular antigen-binding region (composed of light and heavy chains derived from monoclonal antibodies joined by a tough hinge region to form single-chain antibodies), a transmembrane region, and an intracellular signal transduction region.The CAR structure is obtained by genetically recombining the scFv that recognizes tumor-associated antigens and the intracellular signal domain "immunoreceptor tyrosine activation motif" in vitro.

The term "immune cell" includes cells that have hematopoietic origin and play a role in immune responses, For example, lymphocytes, such as B cells and T cells; natural killer cells; myeloid cells, such as monocytes, macrophages, eosinophils, mast cells, basophils, and granulocytes.

### Abbreviations

CDR: Complementarity determining region in the variable region of immunoglobulin.
VH: The variable region of the antibody heavy chain.
VL: The variable region of the antibody light chain.
HC: Antibody heavy chain.
LC: Antibody light chain.
IgG: Immunoglobulin G.
AbM: The definition of AbM CDR comes from Martin's related research (Martin ACR, Cheetham JC, Rees AR (1989) Modelling antibody hypervariable loops: A combined algorithm. Proc Natl Acad Sci USA 86: 9268-9272). This definition method integrates some of the definitions of both Kabat and Chothia.Kabat : The immunoglobulin alignment and numbering system proposed by Elvin A. Kabat (see, for example, Kabat et al., Sequences of Proteins of Immunological Interest, 5th Ed. Public Health Service, National Institutes of Health, Bethesda, Md., 1991).
Chothia: The immunoglobulin numbering system proposed by Chothia et al. is a classic rule for identifying the boundaries of CDR regions based on the position of structural loop regions (see, for example, Chothia & Lesk (1987) J. Mol. Biol. 196:901-917; Chothia et al. (1989) Nature 342:878-883).
IMGT: Based on the numbering system of the international ImMunoGeneTics information system ^{®} (IMGT) initiated by Lefranc et al., see Lefranc et al., Dev. Comparat. Immunol. 27:55-77, 2003.
mAb: monoclonal antibody.
EC50: The concentration that produces 50% efficacy or binding.
IC50: The concentration that produces 50% inhibition.
ELISA: Enzyme-linked immunosorbent assay.
PCR: polymerase chain reaction.
HRP: Horseradish peroxidase.
LIFR: Leukemia Inhibitory Factor Receptor.
IL7Rα: Interleukin 7 receptor alpha subunit.
TARC: Thymus activation regulatory chemokine.
hFc: Fc segment of human IgG antibody.
KD: dissociation equilibrium constant.
HCDR1: Complementarity determining region 1 in the variable region of the immunoglobulin heavy chain.
HCDR2: Complementarity determining region 2 in the variable region of the immunoglobulin heavy chain.
HCDR3: Complementarity determining region 3 in the variable region of the immunoglobulin heavy chain.
LCDR1: Complementarity determining region 1 in the variable region of the immunoglobulin light chain.
LCDR2: Complementarity determining region 2 in the variable region of the immunoglobulin light chain.
LCDR3: Complementarity determining region 3 in the variable region of the immunoglobulin light chain.

### Detailed description of the invention

### Antigen binding protein

The present invention prepares a series of fully human monoclonal antibodies. These anti-human LIFR antibodies are prepared by immunizing fully human antibody transgenic mice, molecular biology, and antibody engineering technology, and have fully human antibody amino acid and gene sequences, thereby ensuring the lowest possible immunogenicity when used in humans. Human antibody transgenic mouse technology was first developed by Abgenix (XenoMouse) and Madarex (HuMab mouse) and used for the preparation of fully human antibodies (Lonberg, et al. (1994) Nature.368(6474):856-859, Lonberg), N. and Huszar, D. (1995) Intern. Rev. Immunol. 13:65-93, Harding, F. and Lonberg, N. (1995) Ann. NYAcad. Sci. 764:536-546). The early transgenic mice produced fully human immunoglobulin (Ig), that is, the variable and constant regions of the antibody were all human sequences. Because human Ig Fc does not match the mouse host Fc receptor, the immune response is weak. After antigen immunization, the antibody titer in the serum is very low, which is manifested in the low efficiency of monoclonal antibody preparation.

In recent years, the technology has undergone major innovations and improvements, mainly in the variable regions of the heavy and light chains using the same and similar constant region sequences as the host immunoglobulin (Ig). The present invention uses the transgenic mouse (Harbour H2L2) developed by Harbour BioMed to successfully prepare the LIFR fully human antibody. Harbour transgenic mice have introduced human immunoglobulin variable region genes and rat immunoglobulin constant region genes, and the mouse's own Ig expression is inactivated. The transgenic mice can produce immune response and antibody titer equivalent to normal mice (such as Balb/c) after being immunized with antigen.

In some embodiments, the antigen binding protein provided by the present invention comprises the following complementarity determining regions:
(1) the heavy chain complementarity determining region 1 HCDR1 comprising the amino acid sequence set forth in any one of SEQ ID NO: 1 to SEQ ID NO: 4 or a variant sequence thereof;
(2) the heavy chain complementarity determining region 2 HCDR2 comprising the amino acid sequence set forth in any one of SEQ ID NO: 5 to SEQ ID NO: 15 or a variant sequence thereof;
(3) the heavy chain complementarity determining region 3 HCDR3 comprising the amino acid sequence set forth in any one of SEQ ID NO: 16-SEQ ID NO: 25 or a variant sequence thereof;
(4) the light chain complementarity determining region 1 LCDR1 comprising the amino acid sequence set forth in any one of SEQ ID NO: 26-SEQ ID NO: 32 or a variant sequence thereof
(5) the light chain complementarity determining region 2 LCDR2 comprising the amino acid sequence set forth in any one of SEQ ID NO: 33-SEQ ID NO: 40 or a variant sequence thereof;
(6) the light chain complementarity determining region 3 LCDR3 comprising the amino acid sequence set forth in any one of SEQ ID NO: 41-SEQ ID NO: 45 or a variant sequence thereof;
the variant sequence is a CDR sequence having one or more amino acid substitutions, deletions or additions compared to the CDR of its source.

In some embodiments, the human LIFR antigen binding protein provided by the present invention comprises the following complementarity determining regions:
(1) the heavy chain complementarity determining region 1 HCDR1 comprising the amino acid sequence set forth in GFTFSSYGMX₁ or the amino acid sequence set forth in SEQ ID NO: 4: GFTFSNYAMT, wherein X₁ = H, D or N; and/or
(2) the heavy chain complementarity determining region 2 HCDR2 comprising the amino acid sequence set forth in VIWYDGX₂NKYYX₃DSVKG, the amino acid sequence set forth in VIWFDGSX₄KYYADSVKG, the amino acid sequence set forth in SEQ ID NO: 7: VIWYDGSNKFYADSVRG, the amino acid sequence set forth in SEQ ID NO: 14: TISGSGAFTYYADAVKG, the amino acid sequence set forth in SEQ ID NO: 15: VISGSGFLTYYADAVKG, wherein X₂=N or S, X₃=E, A or T, X₄=N, I, L or V; and/or
(3) the heavy chain complementarity determining region 3 HCDR3 comprising the amino acid sequence set forth in SEQ ID NO: 16: DGESSMVRGLLNWFDP, the amino acid sequence set forth in SEQ ID NO: 17: ELRYFDWLLSPFDY, the amino acid sequence set forth in SEQ ID NO: 21: GERTLDL, the amino acid sequence set forth in ELWFGELLSPX₅DF, the amino acid sequence set forth in GQLVX₆DX₇, or the amino acid sequence set forth in GGILTGFDXs, wherein Xs=L or F, X₆=G or Q, X₇=Y, F or L, X₈=N or Y; and/or
(4) the light chain complementarity determining region 1 LCDRlcomprising the amino acid sequence set forth in RASQSX₉SSSX₁₀LA, the amino acid sequence set forth in RASQSISSX₁₁LX₁₂, or the amino acid sequence set forth in RASQNLNSNLA, wherein X₉=V or I, X₁₀=Y or F, X₁₁=Y, W or N, X₁₂=N or A; and/or
(5) the light chain complementarity determining region 2 LCDR2 comprising the amino acid sequence set forth in GX₁₃SSRAT, the amino acid sequence set forth in KASX₁₄LEX₁₅, the amino acid sequence set forth in SEQ ID NO: 35: AASNRAT, the amino acid sequence set forth in SEQ ID NO: 36: AASSLQS, the amino acid sequence set forth in SEQ ID NO: 40: GASTRAP,wherein X₁₃=A or T, X₁₄=S Or N, X₁₅=S or N; and/or
(6) the light chain complementarity determining region 3 LCDR3 comprising the amino acid sequence set forth in SEQ ID NO: 41: QQYGSSPFT, the amino acid sequence set forth in SEQ ID NO: 42: QQSYSTPLT, the amino acid sequence set forth in SEQ ID NO: 43: QQYKSFSPGGLT, the amino acid sequence set forth in SEQ ID NO: 44: QQYKSNPLT, the amino acid sequence set forth in SEQ ID NO: 45: QQYNNWPRT.

In some embodiments, the antigen binding protein provided by the present invention comprises the following heavy chain variable region VH and light chain variable region VL:
(1) the heavy chain variable region VH comprising the amino acid sequence set forth in any one of SEQ ID NO:46-SEQ ID NO:59; and/or the light chain variable region VL comprising the amino acid sequence set forth in any one of SEQ ID NO:60-SEQ ID NO:69; or
(2) the VH has at least 70%, at least 80%, at least 85%, at least 90%, at least 91%, at least 92%, at least 93%, at least 94%, at least 95%, at least 96%, at least 97%, at least 98%, or at least 99% sequence identity to any one of VH in (1); and/or, the VL has at least 70%, at least 80%, at least 85%, at least 90%, at least 91%, at least 92%, at least 93%, at least 94%, at least 95%, at least 96%, at least 97%, at least 98%, or at least 99% sequence identity to any one of VL in (1); or
(3) the VH has one or more amino acid substitutions, deletions or additions or any combination thereof compared to any one of VH in (1); and/or, the VL has one or more amino acid substitutions, deletions or additions or any combination thereof compared to any one of VL in (1); the substitutions are conservative substitutions.

In some embodiments, the antigen binding protein provided by the present invention comprises the following heavy chain HC and light chain LC:
(1) a heavy chain HC comprising the amino acid sequence set forth in any one of SEQ ID NO: 70-SEQ ID NO: 83; and/or a light chain LC comprising the amino acid sequence set forth in any one of SEQ ID NO: 84-SEQ ID NO: 93; or
(2) the heavy chain has at least 80%, at least 85%, at least 90%, at least 91%, at least 92% , at least 93%, at least 94%, at least 95%, at least 96%, at least 97%, at least 98%, or at least 99% sequence identity to the heavy chain in (1) ; and/or, the light chain has at least 70%, at least 80%, At least 85%, at least 90%, at least 91%, at least 92%, at least 93%, at least 94%, at least 95%, at least 96%, at least 97%, at least 98%, or at least 99% sequence identity to the light chain in (1).

In some specific embodiments, the human LIFR antibody prepared by the present invention comprises the combination of heavy chain and light chain as described in Table 1 below.

**Table 1 Sequence characteristics of antibodies that bind to LIFR protein**

| **SEQ ID NO.** | **PR300516** | **PR301164** | **PR301168** | **PR301176** | **PR301152** |
|---|---|---|---|---|---|
| HCDR 1 | SEQ ID NO: 1 | SEQ ID NO: 1 | SEQ ID NO:2 | SEQ ID NO:2 | SEQ ID NO:2 |
| | GFTFSSYGMH | GFTFSSYGMH | GFTFSSYGMD | GFTFSSYGMD | GFTFSSYGMD |
| HCDR 2 | SEQ ID NO:5 | SEQ ID NO:6 | SEQ ID NO:7 | SEQ ID NO:7 | SEQ ID NO:8 |
| | VIWYDGNNKY YADSVKG | VIWYDGSNKY YEDSVKG | VIWYDGSNKF YADSVRG | VIWYDGSNKF YADSVRG | VIWYDGSNKY YADSVKG |
| HCDR 3 | SEQ ID NO:16 | SEQ ID NO:17 | SEQ ID NO:18 | SEQ ID NO:19 | SEQ ID NO:19 |
| | DGESSMVRGL LNWFDP | ELRYFDWLLS PFDY | ELWFGELLSPL DF | ELWFGELLSPF DF | ELWFGELLSPF DF |
| LCDR 1 | SEQ ID NO:26 | SEQ ID NO:27 | SEQ ID NO:26 | SEQ ID NO:26 | SEQ ID NO:28 |
| | RASQSVSSSYL A | RASQSVSSSFL A | RASQSVSSSYL A | RASQSVSSSYL A | RASQSISSSYL A |
| LCDR 2 | SEQ ID NO:33 | SEQ ID NO:34 | SEQ ID NO:33 | SEQ ID NO:33 | SEQ ID NO:35 |
| | GASSRAT | GTSSRAT | GASSRAT | GASSRAT | AASNRAT |
| LCDR 3 | SEQ ID NO:41 | SEQ ID NO:41 | SEQ ID NO:41 | SEQ ID NO:41 | SEQ ID NO:41 |
| | QQYGSSPFT | QQYGSSPFT | QQYGSSPFT | QQYGSSPFT | QQYGSSPFT |
| VH | SEQ ID NO:46 | SEQ ID NO:47 | SEQ ID NO:48 | SEQ ID NO:49 | SEQ ID NO:50 |
| VL | SEQ ID NO:60 | SEQ ID NO:61 | SEQ ID NO:62 | SEQ ID NO:62 | SEQ ID NO:63 |
| HC | SEQ ID NO:70 | SEQ ID NO:71 | SEQ ID NO:72 | SEQ ID NO:73 | SEQ ID NO:74 |
| LC | SEQ ID NO:84 | SEQ ID NO:85 | SEQ ID NO:86 | SEQ ID NO:86 | SEQ ID NO:87 |

| **SEQ ID NO.** | **PR301160** | **PR301153** | **PR301177** | **PR301195** | **PR301196** |
|---|---|---|---|---|---|
| HCDR 1 | SEQ ID NO:2 | SEQ ID NO: 1 | SEQ ID NO:3 | SEQ ID NO:3 | SEQ ID NO:3 |
| | GFTFSSYGMD | GFTFSSYGMH | GFTFSSYGMN | GFTFSSYGMN | GFTFSSYGMN |
| HCDR 2 | SEQ ID NO:9 | SEQ ID NO:10 | SEQ ID NO:10 | SEQ ID NO:11 | SEQ ID NO:11 |
| | VIWYDGSNKY YTDSVKG | VIWFDGSNKY YADSVKG | VIWFDGSNKY YADSVKG | VIWFDGSIKYY ADSVKG | VIWFDGSIKYY ADSVKG |
| HCDR 3 | SEQ ID NO:19 | SEQ ID NO:20 | SEQ ID NO:21 | SEQ ID NO:22 | SEQ ID NO:22 |
| | ELWFGELLSPF DF | GQLVGDY | GERTLDL | GQLVQDF | GQLVQDF |
| LCDR 1 | SEQ ID NO:26 | SEQ ID NO:29 | SEQ ID NO:30 | SEQ ID NO:30 | SEQ ID NO:30 |
| | RASQSVSSSYL A | RASQSISSYLN | RASQSISSWLA | RASQSISSWLA | RASQSISSWLA |
| LCDR 2 | SEQ ID NO:35 | SEQ ID NO:36 | SEQ ID NO:37 | SEQ ID NO:38 | SEQ ID NO:39 |
| | AASNRAT | AASSLQS | KASSLES | KASNLES | KASNLEN |
| LCDR 3 | SEQ ID NO:41 | SEQ ID NO:42 | SEQ ID NO:43 | SEQ ID NO:44 | SEQ ID NO:44 |
| | QQYGSSPFT | QQSYSTPLT | QQYKSFSPGG LT | QQYKSNPLT | QQYKSNPLT |
| VH | SEQ ID NO:51 | SEQ ID NO:52 | SEQ ID NO:53 | SEQ ID NO:54 | SEQ ID NO:54 |
| VL | SEQ ID NO:64 | SEQ ID NO:65 | SEQ ID NO:66 | SEQ ID NO:67 | SEQ ID NO:68 |
| HC | SEQ ID NO:75 | SEQ ID NO:76 | SEQ ID NO:77 | SEQ ID NO:78 | SEQ ID NO:78 |
| LC | SEQ ID NO:88 | SEQ ID NO:89 | SEQ ID NO:90 | SEQ ID NO:91 | SEQ ID NO:92 |

| **SEQ ID NO.** | **PR301211** | **PR301215** | **PR301218** | **PR301127** | **PR301172** |
|---|---|---|---|---|---|
| HCDR 1 | SEQ ID NO:1 | SEQ ID NO:1 | SEQ ID NO:1 | SEQ ID NO:4 | SEQ ID NOT |
| | GFTFSSYGMH | GFTFSSYGMH | GFTFSSYGMH | GFTFSNYAMT | GFTFSNYAMT |
| HCDR 2 | SEQ ID NO:12 | SEQ ID NO:11 | SEQ ID NO:13 | SEQ ID NO:14 | SEQ ID NO:15 |
| | VIWFDGSLKY YADSVKG | VIWFDGSIKYY ADSVKG | VIWFDGSVKY YADSVKG | TISGSGAFTYY ADAVKG | VISGSGFLTYY ADAVKG |
| HCDR 3 | SEQ ID NO:23 | SEQ ID NO:22 | SEQ ID NO:22 | SEQ ID NO:24 | SEQ ID NO:25 |
| | GQLVQDL | GQLVQDF | GQLVQDF | GGILTGFDN | GGILTGFDY |
| LCDR 1 | SEQ ID NO:30 | SEQ ID NO:30 | SEQ ID NO:30 | SEQ ID NO:31 | SEQ ID NO:32 |
| | RASQSISSWLA | RASQSISSWLA | RASQSISSWLA | RASQSISSNLA | RASQNLNSNL A |
| LCDR 2 | SEQ ID NO:38 | SEQ ID NO:38 | SEQ ID NO:38 | SEQ ID NO:37 | SEQ ID NO:40 |
| | KASNLES | KASNLES | KASNLES | KASSLES | GASTRAP |
| | SEQ ID NO:44 | SEQ ID NO:44 | SEQ ID NO:44 | SEQ ID NO:43 | SEQ ID NO:45 |
| LCDR 3 | QQYKSNPLT | QQYKSNPLT | QQYKSNPLT | QQYKSFSPGG LT | QQYNNWPRT |
| VH | SEQ ID NO:55 | SEQ ID NO:56 | SEQ ID NO:57 | SEQ ID NO:58 | SEQ ID NO:59 |
| VL | SEQ ID NO:67 | SEQ ID NO:67 | SEQ ID NO:67 | SEQ ID NO:66 | SEQ ID NO:69 |
| HC | SEQ ID NO:79 | SEQ ID NO:80 | SEQ ID NO:81 | SEQ ID NO:82 | SEQ ID NO:83 |
| LC | SEQ ID NO:91 | SEQ ID NO:91 | SEQ ID NO:91 | SEQ ID NO:90 | SEQ ID NO:93 |

The human LIFR antibodies shown in Table 1 are analyzed by Mega software for sequence similarity, which can be divided into the following five groups:
Group 1: PR300516;
Group 2: PR301164, PR301168, PR301176;
Group 3: PR301152, PR301160;
Group 4: PR301153, PR301177, PR301195, PR301196, PR301211, PR301215, PR301218;
Group 5: PR301127, PR301172.

In some embodiments, PR300516, PR301127, PR301152, PR301168, and PR301211 are selected from the five groups ofantibodies for experiments.

### Antibody derivatives and multispecific antibodies

The antigen-binding proteins provided by the present invention as described in the above section comprise antibodies or antigen-binding fragments, which can be derivatized (for example, linked to another molecule, such as another polypeptide or protein). Generally, the derivatization (for example, labeling) of the antibody or its antigen-binding fragment will not adversely affect its binding to LIFR (especially human LIFR). Therefore, the antibodies or antigen-binding fragments thereof of the present invention are also intended to include such derivatized forms. For example, the antibody or antigen-binding fragment of the present invention can be linked to one or more other molecular groups to form a bispecific antibody, detection reagent, pharmaceutical reagent, and/or proteins or peptides capable of mediating the binding of an antibody or antigen-binding fragment to another molecule (for example, avidin or polyhistidine tag).

One type of derivatized antibody is a labeled antibody. For example, the antibody or antigen-binding fragment thereof of the present invention can be linked to a detectable label. The detectable label of the present invention can be any substance that can be detected by fluorescence, spectroscopy, photochemistry, biochemistry, immunology, electrical, optical, or chemical means. Such labels are well known in the art, examples of which include, but are not limited to, enzymes (for example, horseradish peroxidase, alkaline phosphatase, β-galactosidase, urease, glucose oxidase, etc.), radioactive nuclear Fluorescein (for example, 3H, 125I, 35S, 14C or 32P), fluorescent dyes (for example, fluorescein isothiocyanate (FITC), fluorescein, tetramethylrhodamine isothiocyanate (TRITC), phycoerythrin ( PE), Texas Red, Rhodamine, quantum dots or cyanine dye derivatives (such as Cy7, Alexa 750), acridine ester compounds, magnetic beads (such as Dynabeads^{®}), calorimetric markers such as colloids Gold or colored glass or plastic (for example, polystyrene, polypropylene, latex, etc.) beads, and biotin for binding avidin (for example, streptavidin) modified by the above-mentioned label.Patents teaching the use of this marker include, but are not limited to, U.S. Patent Nos. 3,817,837; 3,850,752; 3,939,350; 3,996,345; 4,277,437; 4,275,149; and 4,366,241 (all incorporated herein by reference).The detectable label as described above can be detected by methods known in the art. For example, a radioactive label can be detected using photographic film or a scintillation calculator, and a fluorescent label can be detected using a light detector to detect the emitted light. Enzyme markers are generally detected by providing a substrate to the enzyme and detecting the reaction product produced by the action of the enzyme on the substrate, and calorimetric markers are detected by simply visualizing colored markers. In certain embodiments, such labels can be suitable for immunological detection (eg, enzyme-linked immunoassay, radioimmunoassay, fluorescent immunoassay, chemiluminescence immunoassay, etc.). In certain embodiments, the detectable label as described above can be connected to the antibody or antigen-binding fragment thereof of the present invention through linkers of different lengths to reduce potential steric hindrance.

In addition, the antibody or antigen-binding fragment thereof of the present invention can also be derivatized with chemical groups, such as polyethylene glycol (PEG), methyl or ethyl, or sugar groups. These groups can be used to improve the biological properties of antibodies, such as increasing serum half-life.

As a derivative of another antibody, the present invention provides a multispecific antibody comprising a first antibody or fragment thereof, and another antibody or fragment thereof, or antibody analogue, wherein the first antibody or fragments thereof, additional antibodies or fragments thereof, or antibody analogs retain their original binding specificity. The first antibody or fragment thereof is any LIFR-binding (monoclonal) antibody or antigen-binding fragment thereof of the present invention. As used herein, "antibody mimetic" refers to the same specific binding to an antigen as an antibody, but without the structure of an antibody. They are usually artificial peptides or proteins with a molar mass of about 3 to 20 kDa, such as ankyrin repeat protein (DARPin) and fynomer. The designed ankyrin repeat protein (DARPin) can be linked to IgG antibody, scFv-Fc antibody fragment or a combination thereof, as described in CN104341529A. The anti-IL-17a fynomer is fused with an anti-IL-6R antibody to produce a bispecific fusion polypeptide, as described in WO2015141862A1.

In certain embodiments, the multispecific antibody is formed by coupling a first antibody or antigen-binding fragment thereof with other antibodies or antigen-binding fragments or antibody analogs thereof, and wherein each antibody or antigen-binding fragment or antibody or the analog retains its original binding specificity, and the first antibody or antigen-binding fragment thereof is the antibody or antigen-binding fragment thereof according to the present invention. In certain embodiments, the multispecific antibody is a bispecific antibody or a trispecific antibody or a tetraspecific antibody.

### Antibody Drug Conjugate

The antibody-drug conjugate comprises the antigen-binding protein moiety provided by the present invention and the conjugated moiety. The antigen binding protein moiety includes the "antigen binding protein" as described above, and the antigen binding fragment obtained based on the "antigen binding protein".

Examples of antigen-binding fragments include, but are not limited to, Fab, Fab', Fv fragment, F(ab')2, scFv, di-scFv and/or dAb and the like. The conjugated moiety may contain at least one drug loaded. The loaded drug may include the active ingredient of the drug and/or the labeling molecule. Loaded drugs are small molecular compounds or toxins or other drug molecular forms with pharmacological activity, which can be but not limited to small molecular compounds, toxin molecules, oligonucleotides, protein degradation targeting chimeras (PROTAC), affinity Ligands, fluorescent groups, nuclide groups, etc. Various drug loads are known in the art. For example, small molecule compounds usually refer to a class of substances with strong cytotoxicity. Exemplary small molecule compounds can exert such cytotoxic and cytostatic effects through mechanisms including but not limited to tubulin binding, DNA binding, RNA polymerase inhibition, protein synthesis, or topoisomerase inhibition. For example, the small molecule compound may be a tubulin inhibitor; for example, the tubulin inhibitor may be maytansine (for example, DM1 or DM4), auristatin (for example, MMAE or MMAF), and so on. For example, the small molecule compound may be a DNA damaging agent; for example, the DNA damaging agent may be calicheamicins, pyrrolobenzodiazepines (PBD), and the like. For example, the protein degradation targeting chimera (PROTAC) is a class of compounds that can induce the degradation of the target protein by inducing the polyubiquitination of the target protein; for example, the PROTAC may be a BET protein degrading agent. The drug conjugate may also comprises at least one linker. For example, the linker may include a cleavable linker or a non-cleavable linker. The linker is used to connect one or more drug loads to the antigen binding protein. In this application, the chain-scissible linker may be a "cleavable" linker that facilitates drug release. For example, cleavable linkers may include, but are not limited to, acid-sensitive linkers, protease-sensitive linkers, light-sensitive linkers, or disulfide-containing linkers. The linker may include one or more linker components, and a variety of linker members are known in the art, for example, maleimidocaproyl (MC, maleimidocaproyl), maleimidocaproyl (MP), Val-citrulline (val-cit or vc, valine-citrulline), paminobenzyloxycarbonyl (PAB, paminobenzyloxycarbonyl).

### Preparation of nucleic acid, vector, host cell and antibody

The antigen binding protein of the present invention can be prepared by various methods known in the art, for example, obtained by genetic engineering recombination technology. For example, DNA molecules encoding the heavy chain and light chain genes of the antibody of the present invention are obtained by chemical synthesis or PCR amplification. The resulting DNA molecule is inserted into the expression vector and then transfected into the host cell. Then, the transfected host cell is cultured under specific conditions, and the antibody of the present invention is expressed.

In certain specific embodiments, the present invention provides isolated nucleic acid molecules comprising a nucleotide sequence encoding an antibody of the present invention or an antigen-binding fragment thereof, or a heavy chain variable region and/or a light chain variable region thereof, or one or more CDRs thereof.According to the codon degeneracy known in the art, in some embodiments, the nucleotide sequence can be replaced based on the codon degeneracy. In certain embodiments, the nucleotide sequence is codon optimized.

In certain embodiments, the invention provides cloning vectors or expression vectors comprising the isolated nucleic acid molecules of the invention. In some embodiments, the vector is, for example, a plasmid, cosmid, phage, lentivirus, and the like. In some embodiments, the vector can express the antibody or antigen-binding fragment thereof of the present invention in a subject (such as a mammal, such as a human). In certain embodiments, the invention provides a host cell comprising the isolated nucleic acid molecule of the invention or the vector of the invention. The host cell may be a eukaryotic cell (for example, a mammalian cell, an insect cell, a yeast cell) or a prokaryotic cell (for example, Escherichia coli). Suitable eukaryotic cells include, but are not limited to, NS0 cells, Vero cells, Hela cells, COS cells, CHO cells, HEK293 cells, BHK cells, and MDCKII cells. Suitable insect cells include but are not limited to Sf9 cells. In certain embodiments, the host cell of the present invention is a mammalian cell, such as CHO (e.g., CHO-K1, CHO-S, CHO DXB 11, CHO DG44).

In certain embodiments, the present invention provides a method for preparing the antibody or antigen-binding fragment thereof of the present invention, which comprises culturing the host cell of the present invention under conditions that allow the expression of the antibody or antigen-binding fragment thereof, and the antibody or antigen-binding fragment thereof is recovered from the culture of the cultured host cell.

### Pharmaceutical use, treatment method, pharmaceutical composition, and drug delivery device

The present invention provides the use of the human LIFR antigen binding protein, antigen binding protein derivative, multispecific antibody, immune cell, antibody-drug conjugate in the preparation of LIF and/or LIFR blocking drugs, preparation for prevention and / or treating LIF and/or LIFR positive diseases.

Correspondingly, the present invention provides the method of the human LIFR antigen binding protein, antigen binding protein derivative, multispecific antibody, immune cell, antibody-drug conjugate for blocking LIF and/or LIFR, for preventing and/ or treatment of LIF and/or LIFR positive diseases.

In some embodiments, the present invention provides a pharmaceutical composition for treatment, the pharmaceutical composition comprises the human LIFR antigen binding protein, nucleic acid molecule, vector, host cell, immune cell, Antigen-binding protein derivatives, multispecific antibodies and/or antibody-drug conjugates and optionally pharmaceutically acceptable carriers. "Pharmaceutically acceptable carriers" include, but are not limited to, diluents, excipients, fillers, wetting agents, disintegrants, flavoring agents, and binders.

In some embodiments, the pharmaceutical composition further includes a therapeutic agent for combinational use, which includes but is not limited to a chemotherapeutic agent, a radiotherapy agent, an immunosuppressive agent, and a cytotoxic drug.

In certain embodiments, in the pharmaceutical composition, the antigen binding protein of the present invention and the therapeutic agent are provided as separate components or as components of the same composition. Therefore, the antigen binding protein of the present invention and the therapeutic agent can be combined or separately administered simultaneously or sequentially. Anti-LIFR antibodies or antigen-binding fragments thereof can be administered alone or in combination with other therapeutic agents. The anti-LIFR antibody or antigen-binding fragment thereof and one or more other therapeutic agents can be administered separately, simultaneously, or sequentially.

The pharmaceutical composition can be administered to the patient in any suitable form (depending on the desired method of administering it to the patient), and can be administered to patients by a variety of routes (e.g., orally, transdermally, subcutaneously, intranasally, intravenously, intramuscularly, intraocularly, locally, intrathecally, and intracerebroventricularly).The most suitable route of administration in any given situation will depend on the specific antibody, the nature and severity of the individual and the disease, and the individual's physical condition.

In some embodiments, the present invention also provides a drug delivery device for administering the antigen binding protein, antigen binding protein derivative, multispecific antibody, immune cell, antibody-drug conjugate, and pharmaceutical composition containing the foregoing components. The device includes:
(i) An infusion module, which is used to administer a pharmaceutical composition comprising an active ingredient to a subject;
(ii) A pharmaceutical composition for infusion, which contains an active ingredient selected from the group consisting of antigen binding protein, antigen binding protein derivative, multispecific antibody, immune cell, antibody-drug conjugates or combinations thereof; and
(iii) Optional drug efficacy monitoring module.

In another preferred embodiment, the administration includes parenteral administration and parenteral administration. In another preferred embodiment, the parenteral administration includes injection administration, and the injection route used includes intravenous, intramuscular, intraarterial, intramembranous, intracapsular, intraorbital, intracardiac, intradermal, intraperitoneal, transtracheal, subcutaneous, subepidermal, intraarticular, subcapsular, subarachnoid, intraspinal, supradural, and intrapleural injections and push-ins. In another preferred embodiment, the parenteral administration includes topical, epidermal, or mucosal administration, such as intranasal, oral, vaginal, rectal, sublingual, or topical application. In another preferred embodiment, the infusion module is a needle-free hypodermic injection device, a micro-infusion pump, a transdermal drug delivery device, a bolus injection device, or an osmotic device.

### Detection method, kit

The antibody or antigen-binding fragment thereof of the present invention can bind to LIFR, and thus can be used to detect the presence or level of LIFR in a sample.

In one aspect, the invention provides a kit comprising the antigen binding protein of the invention. In certain embodiments, the antigen binding protein of the invention includes a detectable label. In a preferred embodiment, the kit further includes a second antibody, which specifically recognizes the antibody of the present invention or an antigen-binding fragment thereof. Preferably, the second antibody further includes a detectable label.

In the present invention, the detectable label may be any substance that can be detected by fluorescence, spectroscopy, photochemistry, biochemistry, immunology, electrical, optical, or chemical means. It is particularly preferable that such a label can be applied to immunological detection (for example, enzyme-linked immunoassay, radioimmunoassay, fluorescence immunoassay, chemiluminescence immunoassay, etc.).

In the present invention, the detection of LIFR expression is provided, which comprises contacting a biological sample (individual cells, tissues, or body fluids) with one or more human LIFR antibodies of the present invention (optionally attached to a detectable part), and whether the test sample is positive for LIFR expression, or whether the test sample has altered (for example, decreased or increased) expression compared to the control sample. In certain embodiments, the tissue or body fluid is peripheral blood, peripheral blood leukocytes, biopsy tissue (eg, lung or skin biopsy), and tissue. The method can be used for diagnostic purposes or non-diagnostic purposes (for example, for LIF/LIFR pathway research, drug screening, histochemical analysis, etc.). In certain embodiments, the sample used for non-diagnostic purposes is a cell sample, such as a cell line or an ex vivo cell culture.

In one embodiment, the present invention provides a method for detecting the presence or level of LIFR in a sample, the method comprising under conditions that allow the formation of a complex between the antibody or antigen-binding fragment thereof of the present invention and LIFR, contacting the sample with the antibody or antigen-binding fragment thereof, and detecting the formation of the complex.

In another aspect, the present invention provides a method for diagnosing LIFR-positive disease in a subject, comprising contacting the antibody or antigen-binding fragment, multispecific antibody, or antibody-drug conjugate of the present invention with subject's sample under conditions that allow the formation of a complex between the antibody or its antigen-binding fragment and LIFR. The formation of the complex is detected, wherein an increase in the level of LIFR indicates the presence of cancer compared with healthy controls.

Optionally, the subject is a mammal, including non-human mammals and humans. Preferably, the subject is a human.

Preferably, the cancer is cholangiocarcinoma, colorectal cancer, glioma, prostate cancer, ovarian cancer, gastric cancer, nasopharyngeal cancer, breast cancer, bladder cancer, pancreatic cancer, non-small cell lung cancer.

In another aspect, the present invention provides a detection kit, which contains the human LIFR antigen binding protein, antigen binding protein derivative of the present invention. In a preferred embodiment, the kit contains an instruction describing its use.

### Example 1 Immunization of Harbour Transgenic Mice

1.Immunogen: The extracellular region (1-833 a.a.) of recombinant human LIFR protein (GenBank accession number: NP_001121143.1) was cloned into pcDNA3.1 vector containing his tag to prepare a recombinant expression plasmid. Transfect HEK293 cells transiently and expand the culture. After 4 days, the cell culture medium was collected, and the culture supernatant was purified to obtain high-purity huLIFR-ECD protein (>90%), which has biological activity, and cryopreserved in- 80°C.
2.Harbour H2L2 mouse immunization: huLIFR-ECD protein is used to immunize 6-8 weeks Harbour H2L2 transgenic mice. All transgenic mice are raised in an SPF environment. In the initial immunization, 50µg of huLIFR-ECD protein was emulsified with Freund's complete adjuvant (Sigma, F5881), and then injected into the mouse intraperitoneally. For the subsequent booster immunization, 25µg of huLIFR-ECD protein was mixed with RIBI adjuvant (Sigma, S6322), and then injected into the mouse intraperitoneally. The interval between each immunization is 2 weeks. Blood was collected 7 days after immunization to detect serum antibody titer by ELISA, and mice with high serum titer were selected for subsequent single B cell screening experiments.

### Example 2 Single B cell screening based on Beacon^{®} Optofluidic system

The Beacon^{®} Optofluidic system uses optical-electric positioning (OEP^{™}) technology to move individual cells. The Beacon photoelectric system is an automated biological instrument that can allow simultaneous biological function tests, experimental analysis, positive clone selection and other operations under cell culture conditions. The Beacon platform can perform these tasks in a massively parallel, automated manner on thousands of cells.

This application uses a plasma cell discovery workflow. In each experiment, up to 14k individual plasma cells can be screened for selection of antibody-secreted antigen-specific plasma cells. Then, these plasma cells secreting specific antibodies were exported separately to 96-well plates containing cell lysates for subsequent single B cell sequencing which can identify the heavy chain and light chain sequence of the antibody produced by a single B cell (monoclonal).

### Example 3 Single B cell sequencing and Preparation of fully human antibodies

The present invention uses a single B cell sequencing method to acquire antibody heavy chain and light chain sequences from a single plasma cell. Single B cell sequencing has become a powerful tool for obtaining antibody sequences. General procedures include purification of RNA from single plasma cell lysate, reverse transcription synthesis of cDNA, amplification and purification of cDNA, amplification of heavy and light chains, cloning and transfection, and Sanger sequencing. The obtained sequences were subjected to uniqueness and clustering analysis, and then the paired heavy- and light-stranded DNA sequences were subjected to plasmid synthesis.Specifically, after obtaining the light and heavy chain variable domain sequences encoding the antibody molecule, the recombinant antibody molecule can be obtained by fusion expression of the light and heavy chain variable structural domain sequences with the corresponding human antibody light and heavy chain constant structural domain sequences using conventional recombinant DNA techniques.In this example, the antibody heavy chain variable domain sequence (VH) was synthesized and cloned into a mammalian cell expression plasmid vector encoding the human IgG1 antibody heavy chain constant domain sequence to encode the full-length heavy chain of the IgG1 antibody. The antibody light chain variable domain sequence (VL) is genetically synthesized and cloned into a mammalian cell expression plasmid vector encoding the human antibody Igκ light chain constant domain sequence to encode the full-length light chain of the antibody. In this example, since the sequence of the variable domain of the anti-LIFR monoclonal antibody molecule obtained from the immunized Harbour H2L2 mouse is a human antibody sequence, this example also obtains a fully human anti-LIFR recombinant IgG1 antibody.

The heavy chain and light chain complementary region determining region (CDR) sequences, heavy chain, and light chain variable region (V), heavy chain and light chain sequences of the human LIFR antibodies obtained in the present invention are as shown in Table 1 above.

The 15 antibodies in Table 1 were analyzed for sequence similarity through Mega software, and it was found that the above antibodies can be divided into 5 groups: (1) Group 1: PR300516; (2) Group 2: PR301164, PR301168, PR301176; (3) Group 3: PR301152, PR301160; (4) Group 4: PR301153, PR301177, PR301195, PR301196, PR301211, PR301215, PR301218; (5) Group 5: PR301127, PR301172. All antibodies were tested in Examples 4.2 and 4.4. Representative antibodies PR300516, PR301127, PR301152, PR301211, and PR301168 were selected from the 5 groups to perform the experiments of Examples 4.1, 4.3, and Example 5. The representative antibody PR300516 and PR3001168 was also tested in vivo PK and pharmacodynamics.

Among the five representative antibodies, the DNA nucleotide sequence of the heavy chain variable region of PR300516 is shown in SEQ ID NO: 94, and the DNA nucleotide sequence of the light chain variable region of PR300516 is shown in SEQ ID NO: 95; the DNA nucleotide sequence of the heavy chain variable region of PR301127 is shown in SEQ ID NO: 96, the DNA nucleotide sequence of light chain variable region of PR301127 is shown in SEQ ID NO: 97; the DNA nucleotide sequence of heavy chain variable region of PR301152 is shown in SEQ ID NO: 98, the DNA nucleotide sequence of the light chain variable region of PR301152 is shown in SEQ ID NO: 99; the DNA nucleotide sequence of heavy chain variable region of PR301168 is shown in SEQ ID NO: 100, the DNA nucleotide sequence of the light chain variable region of PR301168 is shown in SEQ ID NO: 101; the DNA nucleotide sequence of the heavy chain variable region of PR301211 is shown in SEQ ID NO: 102, and the light chain DNA nucleotide sequence of variable region of PR301211 is shown in SEQ ID NO:103.

### Example 4 Expression and purification of recombinant antibodies

293F cells are used for antibody expression. Adjust the cell density to 1×10e6 cells/mL. Add the heavy chain and light chain antibody plasmids to the tube at a ratio of 1:1.5, and then add the transfection reagent PEI. The ratio of PEI to DNA is 4:1. After mixing, incubate at room temperature for 15 minutes. The above mixture was added to the cells, and the transfected cells were cultured in a 37°C, 5% CO2 incubator, shaking at 125 rpm. 24h after transfection, supplement with OPM-CHO PFF05 nutrition (Opmey, catalog number FB1279-001) to a final concentration of 3%. After the cells were cultured for 5 days, when the cell viability fell below 70%, the supernatant was collected. Protein G column was used for protein purification on the transfection supernatant.

### 4.1 Antibody affinity test

The binding kinetics of the five antibodies PR300516, PR301127, PR301152, PR301168, PR301211, obtained according to the method of Examples 1-4 of the present invention, were examined by the Biolayerinterferometry (BLI) method (Fortebio octet RED 96e) for the human LIFR-ECD protein.

Dilute the antibody to be tested to a final concentration of 6µg/mL, directly immobilize it on the AHC biosensor, perform kinetic measurement, dilute the antigen protein (Human LIF) with 0.02% PBST20 to 3 concentrations of 100nM, 50nM, 25nM, respectively, and the sample was loaded for 70 s, the binding time was 300s, the dissociation time was 300s, and 10 mM of glycine-HCI (pH 2.0) was added and regenerated for 15 s. A simple one-to-one Languir binding model (Octet Red 96 data analysis software) was used to calculate the binding rate (kon) and the dissociation rate (kdis) , the equilibrium dissociation constant (kD) is calculated as the ratio kdis/kon. The results of antibody affinity detection are shown in Table 2 below.

**Table 2 Antibody affinity test results**

| Antibody | PR301127 | PR301152 | PR301168 | PR301211 | PR300516 | MSC-1 |
|---|---|---|---|---|---|---|
| KD | - | 1.57E-08 | 4.92E-10 | 4.21E-10 | 3.04E-10 | 1.25E-09 |

It can be seen from Table 2 that the affinity of human LIFR antibody is higher than that of LIF antibody MSC-1.

### 4.2 Detection of the binding ability of antibodies to LIFR-ECD at the protein level

The binding ability of the 15 antibodies prepared in examples 1-4 of the present invention to the LIFR-ECD protein was detected based on the ELISA assay method. The binding affinity of different antibodies was determined by comparing their dose response binding curves to LIFR-ECD protein. The specific experimental process is as follows: First, human LIFR-ECD, monkey LIFR-ECD and mouse LIFR-ECD proteins are diluted to a concentration of 1µg/mL, added to a 96-well plate (100µL per well) and placed at 4°C overnight. The 96-well plates were washed three times with PBST solution and then placed at 37°C for 1h with the addition of 2% BSA in PBS solution. Antibody seris dilution is prepared and (100nM, 10-fold-dilution), added to a 96-well plate and incubate at 37°C for 1h. After washing three times with PBST solution, add anti-human IgG Fc-HRP secondary antibody (for 5000x dilution) and incubate at 37°C for 30-60min. After washing three times with PBST solution, add TMB color development solution for 5-15 minutes, and then add termination solution to ternimate the color development.

**Table 3 Test results of the binding of antibodies to LIFR-ECD at the protein level**

| | | | | | | | |
|---|---|---|---|---|---|---|---|
| Human LIFR-ECD | Antibody | PR300516 | PR301127 | PR301172 | PR301152 | PR301153 | hIgG1 |
| | EC50 | 0.012 | 0.039 | 0.263 | 0.011 | 0.023 | 92.80 |
| | Antibody | PR301160 | PR301164 | PR301168 | PR301176 | PR301177 | - |
| | EC50 | 0.018 | 0.015 | 0.014 | 0.014 | 0.011 | - |
| | Antibody | PR301195 | PR301196 | PR301211 | PR301215 | PR301218 | - |
| | EC50 | 0.012 | 0.011 | 0.014 | 0.017 | 0.017 | - |
| Monkey LIFR-ECD | Antibody | PR300516 | PR301127 | PR301172 | PR301152 | PR301153 | hIgG1 |
| | EC50 | 0.015 | 0.04769 | 0.191 | 0.017 | 0.033 | ∼10.75 |
| | Antibody | PR301160 | PR301164 | PR301168 | PR301176 | PR301177 | - |
| | EC50 | 0.019 | 0.016 | 0.016 | 0.020 | 0.013 | - |
| | Antibody | PR301195 | PR301196 | PR301211 | PR301215 | PR301218 | - |
| | EC50 | 0.014 | 0.013 | 0.012 | 0.015 | 0.014 | - |
| Mouse LIFR-ECD | Antibody | PR300516 | PR301127 | PR301172 | PR301152 | PR301153 | hIgG1 |
| | EC50 | NA | 0.020 | 0.198 | NA | NA | 4.888 |
| | Antibody | PR301160 | PR301164 | PR301168 | PR301176 | PR301177 | - |
| | EC50 | NA | NA | NA | NA | NA | - |
| | Antibody | PR301195 | PR301196 | PR301211 | PR301215 | PR301218 | - |
| | EC50 | NA | NA | 17.900 | NA | NA | - |

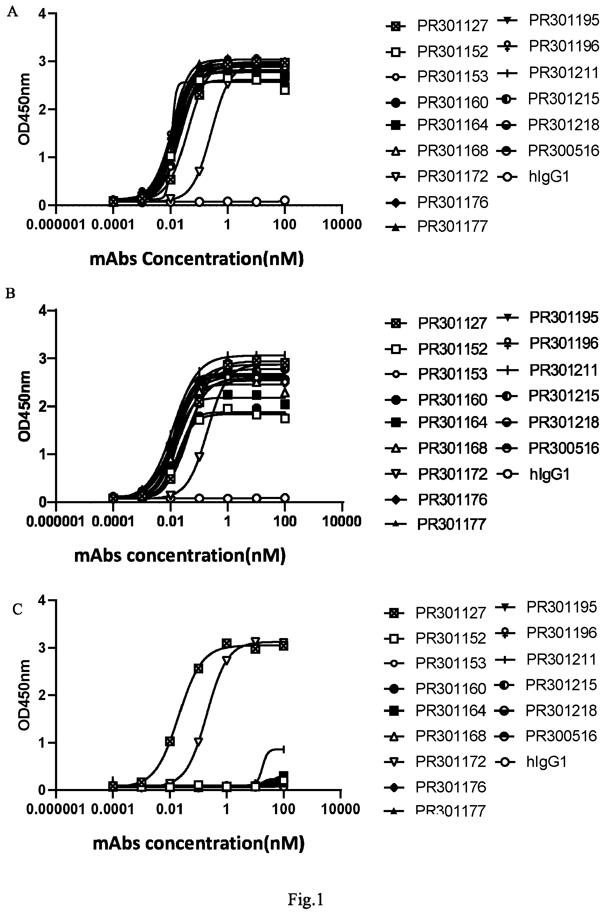

The test results are shown in Table 3 and Fig.1. PR300516, PR301127, PR301172, PR301152, PR301153, PR301160, PR301164, PR301168, PR301176, PR301177, PR301195, PR301196, PR301211, PR301215 and PR301218 antibodies all have binding ability to bind to human LIFR-ECD protein and monkey LIFR-ECD protein, but only PR301127 and PR301172 antibodies can cross-binding to mouse LIFR-ECD protein.

### 4.3 Detection of the ability of antibodies to bind to LIFR and LIFR/gp130 at the cellular level

The flow cytometry assay was used to detect the binding affinity of the five antibodies PR300516, PR301127, PR301152, PR301168, and PR301211 prepared in examples 1-4 to human LIFR expressed on the surface of 293T cells or human LIFR/gp130 heterodimer. The binding ability of different antibodies was determined by comparing the binding curves of human LIFR expressed on the surface of 293T cells or human LIFR/gp130 heterodimer.

The specific experimental process is: (1) Use lenti-huLIFR and lenti-LIFR/gp130 lentivirus to infect HEK293T cells, screen the infected cells with puromycin to obtain a stable cell pool: HEK293T-huLIFR and HEK293T-huLIFR/gp130; (2 ) Perform subclone screening on the stable cell pool to obtain a monoclonal cell line that highly expresses the target gene; (3) make series dilution of test antibody and incubate with the monoclonal cell line at 4°C for 30 minutes, and wash three times with PBS , use FITC-labeled goat anti-human secondary antibody to incubate with 4°C for 30min, wash with PBS three times again, resuspend the cells in 100µL FACS buffer; (4) The EC50 and the peak of the curve were compared using the logarithm of the antibody concentration with a base of 10 as the horizontal coordinate and the median fluorometric value of one channel as the vertical coordinate.

**Table 4 Test results of the ability of antibodies to bind to LIFR and LIFR/gp130 at the cellular level**

| Cell | Value | PR301127 | PR301152 | PR301168 | PR301211 | PR300516 | hIgG1 |
|---|---|---|---|---|---|---|---|
| HEK293T-huLIFR | EC50 | - | 1.465 | 1.200 | 1.106 | 1.016 | - |
| | Span | 237 | 20058 | 19377 | 15875 | 19815 | 11.96 |
| HEK293T-huLIFR/gp130 | EC50 | 79.17 | 0.6562 | 0.4816 | 0.4513 | 0.3615 | 73.78 |
| | Span | 55.63 | 7208 | 6954 | 5800 | 6878 | 90.07 |

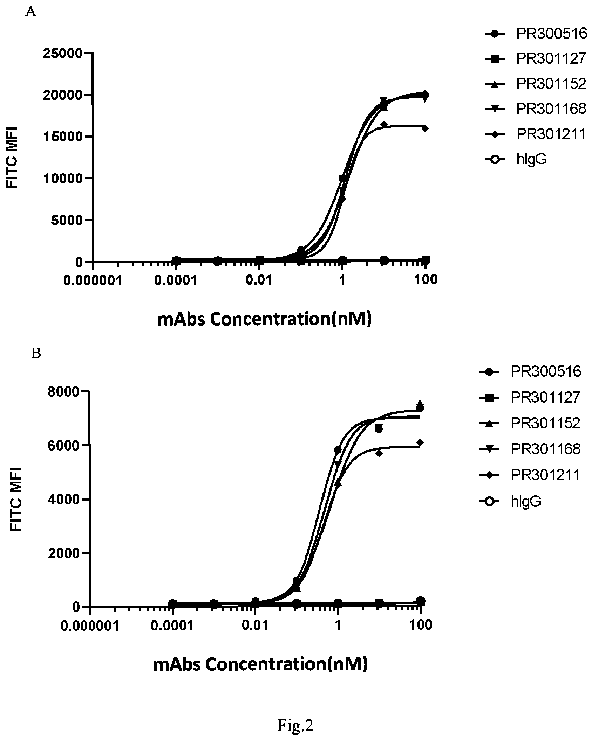

The test results are shown in Table 4 and Fig.2. Under the same concentration conditions, human LIFR antiobody binding to HEK293T-huLIFR/gp130 cells is stronger than that of HEK293T-huLIFR cells.

### 4.4 Detection of the ability of antibodies to block the binding of LIF to LIFR/gp130

The ability of the 15 antibodies prepared in examples 1-4 of the present invention to block the binding of the huLIF protein to the human LIFR/gp130 heterodimer expressed on the surface of 293T cells was tested based on flow cytometry. The blocking ability was determined by comparing the dose response curves of different antibodies blocking the binding of huLIF protein to HEK293T-huLIFR/gp130 cells.

The specific experimental process is: (1) First determine the EC80 concentration of huLIF protein binding to HEK293T-huLIFR/gp 130 cells. Label the huLIF protein to obtain the huLIF-biotin protein; (2) make serise diluton of test antibodies and incubate with HEK293T-huLIFR/gp130 cells and huLIF protein (final concentration is the value of EC80) at 4°C. After washing three times with PBS, incubate with APC-labeled Strepavidin secondary antibody for 30 minutes at 4°C. After washing three times with PBS, resuspend the cells with 100 µL FACS buffer; (3) Use flow cytometry to measure the four-channel median fluorescence Degree value. Use the logarithm of the antibody concentration to base 10 as the abscissa, and plot the four-channel median fluorescence value on the ordinate to compare the IC50 and the peak value of the curve.

**Table 5 Test results of the ability of antibodies to block the binding of LIF to LIFR/gp130**

| Antibody | PR300516 | PR301127 | PR301172 | PR301152 | PR301153 | hIgG1 |
|---|---|---|---|---|---|---|
| IC50 | 0.562 | NA | NA | 2.018 | 7.382 | 0.001774 |
| Antibody | PR301160 | PR301164 | PR301168 | PR301176 | PR301177 | - |
| IC50 | 2.404 | 1.129 | 0.991 | 1.436 | 0.496 | - |
| Antibody | PR301195 | PR301196 | PR301211 | PR301215 | PR301218 | - |
| IC50 | 0.753 | 0.799 | 0.572 | 0.533 | 0.628 | - |

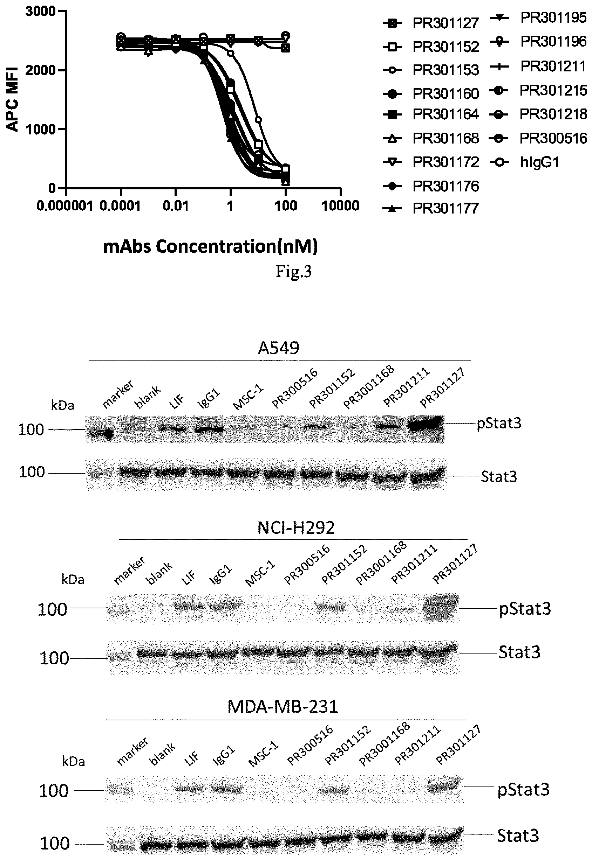

The test results are shown in Table 5 and Fig.3. Compared with the negative control hIgG1, PR300516, PR301152, PR301153, PR301160, PR301164, PR301168, PR301176, PR301177, PR301195, PR301196, PR301211, PR301215 and PR301218 can all significantly block huLIF binding to HEK293T-huLIFR/gp130.

### Example 5 Detection of the inhibitory effect of antibodies on STAT3 signaling pathway

Western blot was used to detect the inhibitory effect of LIF-induced phosphorylation at tyrosine 705 of STAT3, including Capan-2 pancreatic cancer cells, MDA-MB-231 breast cancer cells, A549 and NCI-H292 non-small cell lung cancer cells. The specific experimental process is as follows: first seed the tumor cells on a 6-well plate, 1x106 cells per well, and starve for 24 hours (no serum added to the medium). Then a certain concentration of huLIF protein and the corresponding antibody were incubated for 1 h in a 37°C incubator. The mixture of huLIF protein and antibody was added to the tumor cells and treated in a 37°C incubator for 30 minutes. Asperiate the medium and wash twice with cold PBS. Add 200µL of pre-chilled lysis buffer (premixed with protease inhibitor and phosphatase inhibitor (final concentration 1×) per well. After incubating on ice for 30 min, transfer lysate to EP tube, centrifuge at 12,000 rpm for 10 min. Transfer the supernatant to a new EP tube, take 25 µL of supernatant protein for BCA quantification, add 5×SDS buffer to the rest, and heat it in a water bath at 95°C for 5 minutes. Run SDS-PAGE gel with the protein sample, after transferring the membrane, add block buffer and incubate at room temperature for 1 hour. Primary antibodies Stat3 (124H6) Mouse mAb (Cell Signaling Technology, #9139), Phospho-Stat3 (Tyr705) Antibody (Cell Signaling Technology, #9131) overnight at 4°C. Wash three times with PBST solution for 10 minutes each time. Add secondary antibody AntiMouse IgG (Fc Specific)-Peroxidase (Sigma-Aldrich, A0168), Peroxidase AffiniPure Goat Anti-Rabbit IgG (H+L) (Jackson immune research, 111-035-045) and incubate for 1h at room temperature. After washing three times with PBST, A+B color developing solution was added, and the western result captured by ChemiDoc Imaging System.

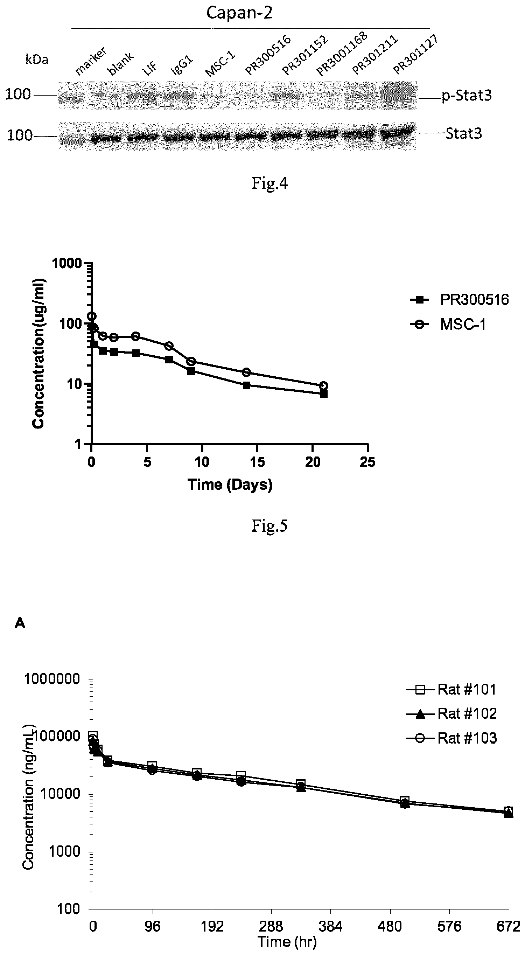

The test results are shown in Fig.4, human LIFR antibody inhibited the activation of STAT3 signaling pathway induced by LIF in four tumor cells, Capan-2 pancreatic cancer cells, MDA-MB-231 breast cancer cells, A549 and NCI-H292 non-small cell lung cancer cells.

### Example 6 PK detection of antibody in vivo

### 6.1 PK in Balb/c mice

The purpose of this study was to determine the pharmacokinetic parameters of female Balb/c mice after intravenous injection of the antibody. The specific experimental process is: a blood sample is taken before the tail vein injection of antibodies in Balb/c mice. Then, after tail vein injection at a dose of 5 mg/kg antibody, blood was taken at 15min, 5h, 24h, day2, day4, day7, day14 and day21 to detect the antibody concentration in the serum. The PK experiment design is shown in Table 6.

**Table 6 PK experimental design**

| Group | Number of mice | Antibody to be tested | Dose (mg/kg ) | Dosing amount (µL/g ) | Mode of administra tion | Dosing plan | Detection time point (N=3/time point) |
|---|---|---|---|---|---|---|---|
| 1 | 6 | MSC-1 (LIF) | 5 | 10 | Intravenou s injection | Single dose | Before administration, single dose 15min, 5h, 24h, 2d, 4d, 7d, 14d, 21d |
| 2 | 6 | PR300516 (LIFR) | 5 | 10 | Intravenou s injection | Single dose | |

**Table 7 PK parameter statistics**

| Antibody | PR300516 | MSC-1 |
|---|---|---|
| T1/2 (hr) | 178.25 | 212.84 |
| Vz (mL/kg) | 114.09 | 81.57 |
| AUCall (hr*µg/mL) | 9521.05 | 16003.13 |
| C1 (mL/hr/kg) | 0.45 | 0.27 |
| C0 (µg/mL) | 92.90 | 134.97 |

It can be seen from Table 7 and Fig.5 that the half-life of the human LIFR antibody PR300516 in mice is 178.25 hours.

### 6.2 PK in Sprague Dawley rats

The purpose of this study was to determine the pharmacokinetic parameters in male Sprague Dawley rats after intravenous injection. The specific experimental process is: Blood samples were taken and retained prior to tail vein injection of antibodies in Sprague Dawley rats. Then, after tail vein injection at the dose of 5mg/kg antibody, blood was taken at 10min, 2h, 8h, 24h, day4, day7, day10, day14, day21 and day28 to detect the antibody concentration in the serum. The PK experiment design is shown in Table 8.

**Table 8 PK experimental design**

| Group | Number of mice | Antibody to be tested | Dose (mg/kg) | Dosing amount (µL/g ) | Mode of administ ration | Dosing plan | Detection time point (N=3/time point) |
|---|---|---|---|---|---|---|---|
| 1 | 3 | PR300516 (LIFR) | 5 | 10 | Intraven ous injection | Single dose | Before administration, single dose |
| | | | | | | | 10min, 2h, 8h, 24h, 4d, 7d, 10d, 14d, 21d and 28d |
| 2 | 3 | PR301168 (LIFR) | 5 | 10 | Intraven ous injection | Single dose | |

**Table 9 PK parameter statistics in rat**

| Antibody | PR300516 | PR301168 |
|---|---|---|
| T1/2 (hr) | 219 | 242 |
| Vz (mL/kg) | 122 | 177 |
| AUCall (hr*µg/mL) | 11056.83 | 8173.60 |
| C1 (mL/hr/kg) | 0.40 | 0.53 |
| C0 (µg/mL) | 92.78 | 65.32 |

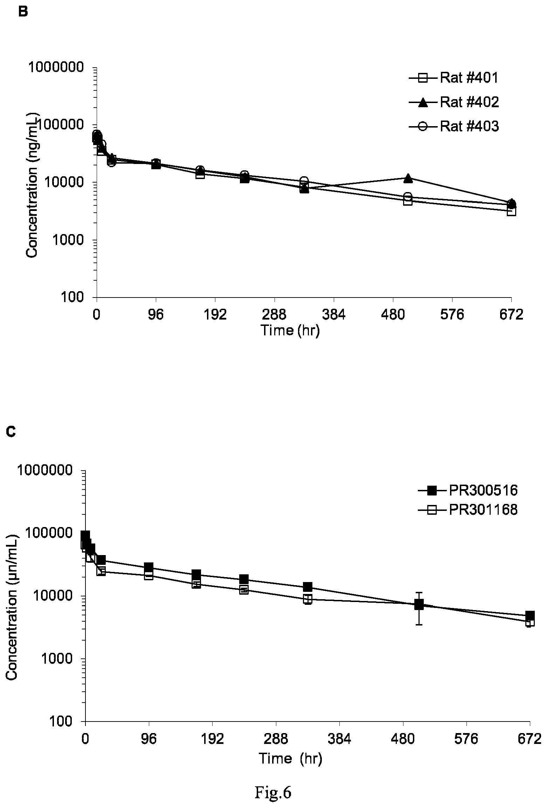

It can be seen from Table 9 and Fig.6 that the half-lives of human LIFR antibodies PR300516 and PR301168 in rats are 219 hours and 242 hours, respectively.

### 3.3 PK in monkey

The purpose of this study was to determine the pharmacokinetic parameters in male and female monkeys after intravenous injection of antibodies. The specific experiment process is as follows: before the tail vein injection of antibodies in male and female monkeys, blood samples are taken first. Then, after tail vein injection at a low dose of 10 mg/kg and a high dose of 100 mg/kg of antibody, the 10mg/kg dosing group will take blood at 10min, 2h, 8h, 24h, day4, day7, day10, and day14 before administration. In the 100mg/kg measurement group, blood was taken at 10min, 2h, 8h, 24h, day4, and day7 in the measurement group to detect the antibody concentration in the serum. The PK experiment design is shown in Table 10.

**Table 10 PK experimental design**

| Group | Number of monkey | Antibody to be tested | Dose (mg/kg ) | Dosing amount (µL/g ) | Mode of administ ration | Dosing plan |
|---|---|---|---|---|---|---|
| 1 | 2 | PR300516 (LIFR) | 10 | Intraven ous injection | Single dose | Before administration, single dose 10min, 2h, 8h, 24h, 2d, 3d, 4d, 7d, 10d, 14d |
| 2 | 2 | PR300516 (LIFR) | 100 | Intraven ous injection | Single dose | |

**Table 11 monkey PK parameter statistics**

| Antibody | | PR300516 | | | |
|---|---|---|---|---|---|
| Dose | | 10 mg/kg | | 100 mg/kg | |
| Animal No | | 101 | 102 | 201 | 202 |
| **T_{1/2}** | hr | 173.71 | 182.90 | 239.27 | 128.24 |
| **Tₘₐₓ** | hr | 0.17 | 0.17 | 0.17 | 0.17 |
| **Cₘₐₓ** | µ g/mL | 258.91 | 243.71 | 2541.99 | 2160.96 |
| **AUCₗₐₛₜ** | hr*µg/mL | 26316.22 | 31817.46 | 187269.17 | 170768.05 |
| **AUC_{INF_obs}** | hr*µg/mL | 35568.46 | 43517.35 | 459474.02 | 318246.46 |
| **V_{z_obs}** | mL/kg | 70.46 | 60.63 | 75.13 | 58.14 |
| **Cl_{_obs}** | mL/hr/kg | 0.28 | 0.23 | 0.22 | 0.31 |

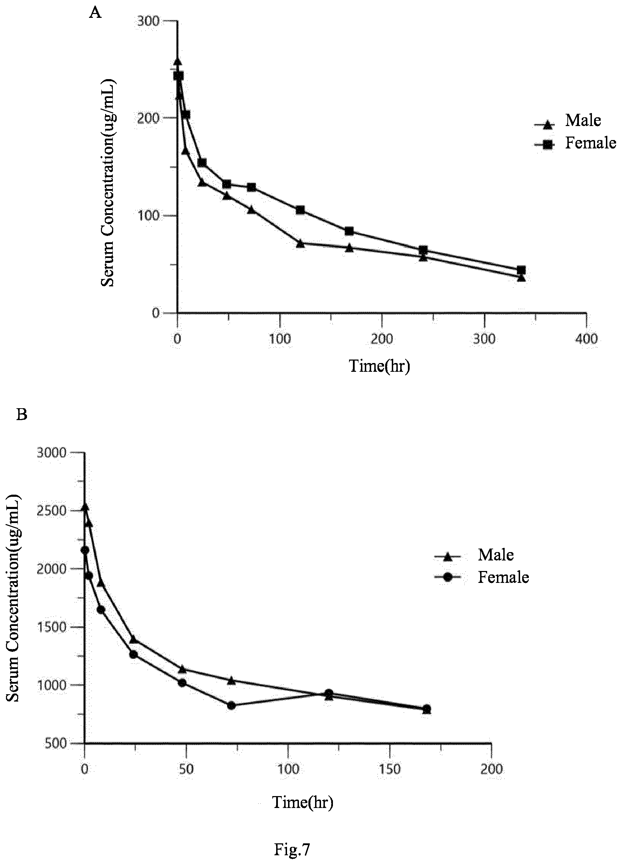

It can be seen from Table 11 and Fig.7 that the half-lives of low-dose human LIFR antibody PR300516 in monkeys are 173.7 hours and 182.9 hours, respectively, and the half-lives of high-dose human LIFR antibody PR300516 in monkeys are 239.3 hours and 128.2 hours, respectively.

### Example 7 Pharmacodynamic experiment of antibody in vivo

### 7.1 Capan-2 model

In this experiment, Capan-2 tumor mouse model was used to study the anti-tumor activity of anti-human LIFR antibody. The pharmacodynamics of PR300516 antibody administered alone (5mg/kg and 15mg/kg) in Capan-2 mouse model were studied. Human pancreatic cancer Capan-2 cells (ATCC cell bank) were subcutaneously inoculated into female Balb/c nude mice (charles river). The treatment method of the antibody group is: on the 5th day, the 9th day, the 12th day, the 16th day, the 19th day and the 23rd day after the tumor cell inoculation, the mice of each group are injected with the corresponding antibodies in the intraperitoneal cavity. The treatment method of the small molecule inhibitor group is: EC359 small molecule inhibitors were administered orally to mice on days 5, 7, 9, 12, 14, 16, 19, 21 and 23 after tumor cell inoculation. The tumor volume was measured on the 5th, 9th, 12th, 16th, 19th and 23rd days after tumor cell inoculation, and then the mice were euthanized.

The results are shown in Table 9 and Fig.6. Compared with the negative control, the anti-human LIFR antibody PR300516 of the present application has a certain inhibition on tumor growth when used alone. Compared to the EC359 small molecule inhibitor and the MSC-1 positive control antibody, the anti-human LIFR antibody described in this application has a stronger inhibition on tumor growth. In this study, mice in all groups showed no significant changes in body weight 37 days after inoculation.

### Capan-1 model

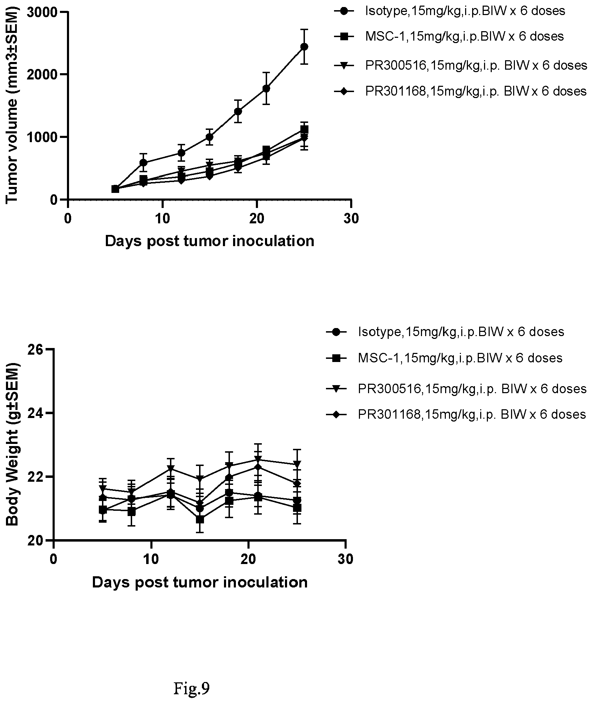

In this experiment, Capan-1 mouse tumor model was used to study the anti-tumor activity of anti-human LIFR antibody. The pharmacodynamics of PR300516 and PR301168 antibodies administered alone (15mg/kg) in the Capan-1 mouse model were studied. Human pancreatic cancer Capan-1 cells (ATCC cell bank) were subcutaneously inoculated into female Balb/c nude mice (charles river). The antibody group treatment method was as follows: on the 5th day, the 8th day, the 12th day, the 15th day, the 18th day and the 21st day after the tumor cell inoculation, the mice of each group were intraperitoneally injected with the corresponding antibody. After tumor cell inoculation on the 5th day, the 8th day, the 12th day, the 15th day, the 18th day, and the 21st day, the tumor volume was measured, and then the mice were euthanized. The results are shown in Table 16 and Fig.9. Compared with the negative control, the anti-human LIFR antibodies PR300516 and PR301168 of the present application have a certain inhibition on tumor growth when used alone. Compared with the MSC-1 positive control antibody, the anti-human LIFR antibody described in the present application has a stronger inhibition on tumor growth. In this study, mice in all groups had no significant changes in their body weight 25 days after inoculation.

### NCI-H292 model

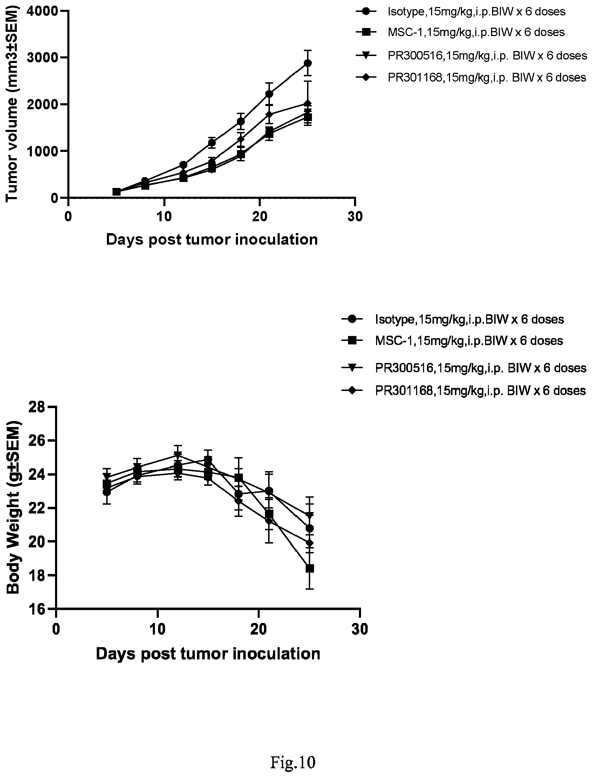

In this experiment, the NCI-H292 mouse tumor model was used to study the anti-tumor activity of anti-human LIFR antibodies. The pharmacodynamics of PR300516 and PR301168 antibodies administered alone (15mg/kg) in the NCI-H292 mouse model were studied. Human pancreatic cancer NCI-H292 cells (ATCC cell bank) were subcutaneously inoculated into female Balb/c nude mice (charles river). The antibody group treatment method was as follows: on the 5th day, the 8th day, the 12th day, the 15th day, the 18th day and the 21st day after the tumor cell inoculation, the mice of each group were intraperitoneally injected with the corresponding antibody. After tumor cell inoculation on the 5th day, the 8th day, the 12th day, the 15th day, the 18th day, and the 21st day, the tumor volume was measured, and then the mice were euthanized. The results are shown in Table 17 and Fig.10. Compared with the negative control, the anti-human LIFR antibodies PR300516 and PR301168 of the present application have a certain inhibition on tumor growth when used alone. Compared with the MSC-1 positive control antibody, the anti-human LIFR antibody described in this application has a slightly weaker inhibition on tumor growth. In this study, the weight of mice in all groups decreased 25 days after inoculation.

**Table 12 Experimental design of antibody in vivo pharmacodynamics (Capan-2)**

| Group | Number of mice | Capan-2(s.c.) DayO | Start administration when the tumor volume 120mm³ | dose (mg/kg) | Mode of administration | Dosing plan |
|---|---|---|---|---|---|---|
| 1 | 6 | 5×10⁶ cell, 100µL PBS+100µL Matrigel (1:1) | Isotype | 5 | Intraperitoneal injection | BIW×6 doses |
| 2 | 6 | | EC359 | 5 | oral | TIW×9 doses |
| 3 | 6 | | EC359 | 15 | oral | TIW×9 doses |
| 4 | 6 | | PR300516 | 5 | Intraperitoneal injection | BIW×6 doses |
| 5 | 6 | | PR300516 | 15 | Intraperitoneal injection | BIW×6 doses |
| 6 | 6 | | MSC-1 | 5 | Intraperitoneal injection | BIW×6 doses |
| 7 | 6 | | MSC-1 | 15 | Intraperitoneal injection | BIW×6 doses |

**Table 13 Experimental design of antibody in vivo pharmacodynamics (Capan-1)**

| Group | Number of mice | Capan-1 (s.c.) DayO | Start administration when the tumor volume 120mm³ | dose (mg/kg) | Mode of administration | Dosing plan |
|---|---|---|---|---|---|---|
| 1 | 6 | 5×10⁶ cell, 100µL PBS+100µL Matrigel (1:1) | Isotype | 15 | Intraperitoneal injection | BIW×6 doses |
| 2 | 6 | | MSC-1 | 15 | Intraperitoneal injection | BIW×6 doses |
| 3 | 6 | | PR300516 | 15 | Intraperitoneal injection | BIW×6 doses |
| 4 | 6 | | PR301168 | 15 | Intraperitoneal injection | BIW×6 doses |

**Table 14 Experimental design of antibody in vivo pharmacodynamics (NCI-H292)**

| Group | Number of mice | NCI-H292 (s.c.) DayO | Start administration when the tumor volume 120mm³ | dose (mg/kg) | Mode of administration | Dosing plan |
|---|---|---|---|---|---|---|
| 1 | 6 | 5×10⁶ cell, 100µL PBS+100µL Matrigel (1:1) | Isotype | 15 | Intraperitoneal injection | BIW×6 doses |
| 2 | 6 | | MSC-1 | 15 | Intraperitoneal injection | BIW×6 doses |
| 3 | 6 | | PR300516 | 15 | Intraperitoneal injection | BIW×6 doses |
| 4 | 6 | | PR301168 | 15 | Intraperitoneal injection | BIW×6 doses |

**Table 15 Statistics of tumor growth inhibition rate in Capan-2 model**

| Group | Dosing | TGI (%) |
|---|---|---|
| 1 | Isotype, 5mg/kg | 0 |
| 2 | EC359, 5mg/kg | 40.99 |
| 3 | EC359, 15mg/kg | 47.85 |
| 4 | PR300516, 5mg/kg | 54.92 |
| 5 | PR300516, 15mg/kg | 55.32 |
| 6 | MSC-1, 5mg/kg | 48.93 |
| 7 | MSC-1, 15mg/kg | 49.34 |

**Table 16 Statistics of tumor growth inhibition rate in Capan-1 model**

| Group | Dosing | TGI (%) |
|---|---|---|
| 1 | Isotype, 15mg/kg | 0 |
| 2 | MSC-1, 15mg/kg | 53.96 |
| 3 | PR300516, 15mg/kg | 59.32 |
| 4 | PR301168, 15mg/kg | 59.89 |

**Table 17 Statistics of tumor growth inhibition rate in NCI-H292 model**

| Group | Dosing | TGI (%) |
|---|---|---|
| 1 | Isotype, 15mg/kg | 0 |
| 2 | MSC-1, 15mg/kg | 39.99 |
| 3 | PR300516, 15mg/kg | 36.35 |
| 4 | PR301168, 15mg/kg | 15.58 |

The above described embodiments only express several embodiments of the present invention, and their descriptions are more specific and detailed, but they are not to be understood as a limitation of the patent scope of the present invention. It should be noted that for a person of ordinary skill in the art, a number of variations and improvements can be made without departing from the conception of the present invention, which all belong to the scope of protection of the present invention. Therefore, the scope of protection of the patent of the present invention shall be subject to the appended claims.

## Claims

1. A human LIFR antigen binding protein comprising the following complementarity determining regions:
(1) the heavy chain complementarity determining region 1 HCDR1 comprising the amino acid sequence set forth in any one of SEQ ID NO: 1 to SEQ ID NO: 4 or a variant sequence thereof;
(2) the heavy chain complementarity determining region 2 HCDR2 comprising the amino acid sequence set forth in any one of SEQ ID NO: 5 to SEQ ID NO: 15 or a variant sequence thereof;
(3) the heavy chain complementarity determining region 3 HCDR3 comprising the amino acid sequence set forth in any one of SEQ ID NO: 16-SEQ ID NO: 25 or a variant sequence thereof;
(4) the light chain complementarity determining region 1 LCDR1 comprising the amino acid sequence set forth in any one of SEQ ID NO: 26-SEQ ID NO: 32 or a variant sequence thereof
(5) the light chain complementarity determining region 2 LCDR2 comprising the amino acid sequence set forth in any one of SEQ ID NO: 33-SEQ ID NO: 40 or a variant sequence thereof;
(6) the light chain complementarity determining region 3 LCDR3 comprising the amino acid sequence set forth in any one of SEQ ID NO: 41-SEQ ID NO: 45 or a variant sequence thereof;
the variant sequence is a CDR sequence having one or more amino acid substitutions, deletions or additions compared to the CDR of its source.

2. The human LIFR antigen binding protein of claim 1, wherein the human LIFR antigen binding protein comprises the following complementarity determining regions:
(1) the heavy chain complementarity determining region 1 HCDR1 comprising the amino acid sequence set forth in GFTFSSYGMX₁ or the amino acid sequence set forth in SEQ ID NO: 4: GFTFSNYAMT, wherein X₁ = H, D or N; and/or
(2) the heavy chain complementarity determining region 2 HCDR2 comprising the amino acid sequence set forth in VIWYDGX₂NKYYX₃DSVKG, the amino acid sequence set forth in VIWFDGSX₄KYYADSVKG, the amino acid sequence set forth in SEQ ID NO: 7: VIWYDGSNKFYADSVRG, the amino acid sequence set forth in SEQ ID NO: 14: TISGSGAFTYYADAVKG, the amino acid sequence set forth in SEQ ID NO: 15: VISGSGFLTYYADAVKG, wherein X₂=N or S, X₃=E, A or T, X₄=N, I, L or V; and/or
(3) the heavy chain complementarity determining region 3 HCDR3 comprising the amino acid sequence set forth in SEQ ID NO: 16: DGESSMVRGLLNWFDP, the amino acid sequence set forth in SEQ ID NO: 17: ELRYFDWLLSPFDY, the amino acid sequence set forth in SEQ ID NO: 21: GERTLDL, the amino acid sequence set forth in ELWFGELLSPX₅DF, the amino acid sequence set forth in GQLVX₆DX₇, or the amino acid sequence set forth in GGILTGFDXs, wherein Xs=L or F, X₆=G or Q, X₇=Y, F or L, X₈=N or Y; and/or
(4) the light chain complementarity determining region 1 LCDRlcomprising the amino acid sequence set forth in RASQSX₉SSSX₁₀LA, the amino acid sequence set forth in RASQSISSX₁₁LX₁₂, or the amino acid sequence set forth in RASQNLNSNLA, wherein X₉=V or I, X₁₀=Y or F, X₁₁=Y, W or N, X₁₂=N or A; and/or
(5) the light chain complementarity determining region 2 LCDR2 comprising the amino acid sequence set forth in GX₁₃SSRAT, the amino acid sequence set forth in KASX₁₄LEX₁₅, the amino acid sequence set forth in SEQ ID NO: 35: AASNRAT, the amino acid sequence set forth in SEQ ID NO: 36: AASSLQS, the amino acid sequence set forth in SEQ ID NO: 40: GASTRAP, wherein X₁₃=A or T, X₁₄=S Or N, X₁₅=S or N; and/or
(6) the light chain complementarity determining region 3 LCDR3 comprising the amino acid sequence set forth in SEQ ID NO: 41: QQYGSSPFT, the amino acid sequence set forth in SEQ ID NO: 42: QQSYSTPLT, the amino acid sequence set forth in SEQ ID NO: 43: QQYKSFSPGGLT, the amino acid sequence set forth in SEQ ID NO: 44: QQYKSNPLT, the amino acid sequence set forth in SEQ ID NO: 45: QQYNNWPRT.

3. The human LIFR antigen binding protein of claim 2, wherein the human LIFR antigen binding protein comprises:
(1) the heavy chain variable region VH comprising the amino acid sequence set forth in any one of SEQ ID NO:46-SEQ ID NO:59; and/or the light chain variable region VL comprising the amino acid sequence set forth in any one of SEQ ID NO:60-SEQ ID NO:69; or
(2) the VH has at least 70%, at least 80%, at least 85%, at least 90%, at least 91%, at least 92%, at least 93%, at least 94%, at least 95%, at least 96%, at least 97%, at least 98%, or at least 99% sequence identity to any one of VH in (1); and/or, the VL has at least 70%, at least 80%, at least 85%, at least 90%, at least 91%, at least 92%, at least 93%, at least 94%, at least 95%, at least 96%, at least 97%, at least 98%, or at least 99% sequence identity to any one of VL in (1); or
(3) the VH has one or more amino acid substitutions, deletions or additions or any combination thereof compared to any one of VH in (1); and/or, the VL has one or more amino acid substitutions, deletions or additions or any combination thereof compared to any one of VL in (1); the substitutions are conservative substitutions.

4. The human LIFR antigen binding protein of claim 3, wherein the human LIFR antigen binding protein comprises:
(1) a heavy chain HC comprising the amino acid sequence set forth in any one of SEQ ID NO: 70-SEQ ID NO: 83; and/or a light chain LC comprising the amino acid sequence set forth in any one of SEQ ID NO: 84-SEQ ID NO: 93; or
(2) the heavy chain has at least 80%, at least 85%, at least 90%, at least 91%, at least 92% , at least 93%, at least 94%, at least 95%, at least 96%, at least 97%, at least 98%, or at least 99% sequence identity to the heavy chain in (1) ; and/or, the light chain has at least 70%, at least 80%, At least 85%, at least 90%, at least 91%, at least 92%, at least 93%, at least 94%, at least 95%, at least 96%, at least 97%, at least 98%, or at least 99% sequence identity to the light chain in (1).

5. The human LIFR antigen binding protein according to any one of claims 1-4, wherein the human LIFR antigen binding protein comprises:
(1) HCDR1 as shown in SEQ ID NO:1, HCDR2 as shown in SEQ ID NO:5, HCDR3 as shown in SEQ ID NO: 16, and/or LCDR1 as shown in SEQ ID NO:26, LCDR2 as shown in SEQ ID NO: 33, LCDR3 as shown in SEQ ID NO: 41; and/or,
(2) HCDR1 as shown in SEQ ID NO: 2, HCDR2 as shown in SEQ ID NO: 7, HCDR3 as shown in SEQ ID NO: 18, and/or LCDR1 as shown in SEQ ID NO: 26, LCDR2 as shown in SEQ ID NO: 33, LCDR3 as shown in SEQ ID NO: 41; and/or,
(3) HCDR1 as shown in SEQ ID NO: 1, HCDR2 as shown in SEQ ID NO: 6, HCDR3 as shown in SEQ ID NO: 17, and/or LCDR1 as shown in SEQ ID NO: 27, LCDR2 as shown in SEQ ID NO: 34, LCDR3 as shown in SEQ ID NO: 41; and/or,
(4) HCDR1 as shown in SEQ ID NO: 2, HCDR2 as shown in SEQ ID NO: 7, HCDR3 as shown in SEQ ID NO: 19, and/or LCDR1 as shown in SEQ ID NO: 26, LCDR2 as shown in SEQ ID NO: 33, LCDR3 as shown in SEQ ID NO: 41; and/or,
(5) HCDR1 as shown in SEQ ID NO: 1, HCDR2 as shown in SEQ ID NO: 12, HCDR3 as shown in SEQ ID NO: 23, and/or LCDR1 as shown in SEQ ID NO: 30, LCDR2 as shown in SEQ ID NO: 38, LCDR3 as shown in SEQ ID NO: 44; and/or,
(6) HCDR1 as shown in SEQ ID NO: 1, HCDR2 as shown in SEQ ID NO: 10, HCDR3 as shown in SEQ ID NO: 20, and/or LCDR1 as shown in SEQ ID NO: 29, LCDR2 as shown in SEQ ID NO: 36, LCDR3 as shown in SEQ ID NO: 42; and/or,
(7) HCDR1 as shown in SEQ ID NO: 3, HCDR2 as shown in SEQ ID NO: 10, HCDR3 as shown in SEQ ID NO: 21, and/or LCDR1 as shown in SEQ ID NO: 30, LCDR2 as shown in SEQ ID NO: 37, LCDR3 as shown in SEQ ID NO: 43; and/or,
(8) HCDR1 as shown in SEQ ID NO: 3, HCDR2 as shown in SEQ ID NO: 11, HCDR3 as shown in SEQ ID NO: 22, and/or LCDR1 as shown in SEQ ID NO: 30, LCDR2 as shown in SEQ ID NO: 38, LCDR3 as shown in SEQ ID NO: 44; and/or,
(9) HCDR1 as shown in SEQ ID NO: 3, HCDR2 as shown in SEQ ID NO: 11, HCDR3 as shown in SEQ ID NO: 22, and/or LCDR1 as shown in SEQ ID NO: 30, LCDR2 as shown in SEQ ID NO: 39, LCDR3 as shown in SEQ ID NO: 44; and/or,
(10) HCDR1 as shown in SEQ ID NO: 1, HCDR2 as shown in SEQ ID NO: 11, HCDR3 as shown in SEQ ID NO: 22, and/or LCDR1 as shown in SEQ ID NO: 30, LCDR2 as shown in SEQ ID NO: 38, LCDR3 as shown in SEQ ID NO: 44; and/or,
(11) HCDR1 as shown in SEQ ID NO: 1, HCDR2 as shown in SEQ ID NO: 13, HCDR3 as shown in SEQ ID NO: 22, and/or LCDR1 as shown in SEQ ID NO: 30, LCDR2 as shown in SEQ ID NO: 38, LCDR3 as shown in SEQ ID NO: 44.

6. The human LIFR antigen binding protein of claim 5, wherein the human LIFR antigen binding protein comprises:
(1) VH as shown in SEQ ID NO: 46, and/or VL as shown in SEQ ID NO: 60;
(2) VH as shown in SEQ ID NO: 48, and/or VL as shown in SEQ ID NO: 62;
(3) VH as shown in SEQ ID NO: 47, and/or VL as shown in SEQ ID NO: 61;
(4) VH as shown in SEQ ID NO: 49, and/or VL as shown in SEQ ID NO: 62;
(5) VH as shown in SEQ ID NO: 55, and/or VL as shown in SEQ ID NO: 67;
(6) VH as shown in SEQ ID NO: 52, and/or VL as shown in SEQ ID NO: 65;
(7) VH as shown in SEQ ID NO:53, and/or VL as shown in SEQ ID NO:66;
(8) VH as shown in SEQ ID NO: 54 and/or VL as shown in SEQ ID NO: 67;
(9) VH as shown in SEQ ID NO: 54 and/or VL as shown in SEQ ID NO: 68;
(10) VH as shown in SEQ ID NO: 56, and/or VL as shown in SEQ ID NO: 67;
(11) VH as shown in SEQ ID NO: 57, and/or VL as shown in SEQ ID NO: 67;
or,the VH has at least 70%, at least 80%, at least 85%, at least 90%, at least 91%, at least 92%, at least 93%, at least 94%, at least 95%, at least 96%, at least 97%, at least 98%, or at least 99% sequence identity compared to any one of VH in (1)-(11); and/or, the VL has at least 70%, at least 80%, at least 85%, at least 90%, at least 91%, at least 92%, at least 93%, at least 94%, at least 95%, at least 96%, at least 97%, at least 98%, or at least 99% sequence identity compared to any one of VL in (1)-(11);
or, the VH has one or more amino acid substitutions, deletions or additions or any combination thereof compared to any one of VH in (1)-(11); and/or, the VL has one or more amino acid substitutions, deletions or additions or any combination thereof compared to any one of VL in (1)-(11); the substitutions are conservative substitutions.

7. The human LIFR antigen binding protein according to claim 6, wherein the human LIFR antigen binding protein comprises:
(1) HC as shown in SEQ ID NO: 70, and/or LC as shown in SEQ ID NO: 84;
(2) HC as shown in SEQ ID NO: 72, and/or LC as shown in SEQ ID NO: 86;
(3) HC as shown in SEQ ID NO: 71, and/or LC as shown in SEQ ID NO: 85;
(4) HC as shown in SEQ ID NO: 73, and/or LC as shown in SEQ ID NO: 86;
(5) HC as shown in SEQ ID NO: 79, and/or LC as shown in SEQ ID NO: 91;
(6) HC as shown in SEQ ID NO: 76, and/or LC as shown in SEQ ID NO: 89;
(7) HC as shown in SEQ ID NO: 77, and/or LC as shown in SEQ ID NO: 90;
(8) HC as shown in SEQ ID NO: 78, and/or LC as shown in SEQ ID NO: 91;
(9) HC as shown in SEQ ID NO: 78, and/or LC as shown in SEQ ID NO: 92;
(10) HC as shown in SEQ ID NO: 80, and/or LC as shown in SEQ ID NO: 91;
(11) HC as shown in SEQ ID NO: 81, and/or LC as shown in SEQ ID NO: 91;
or,the HC has at least 70%, at least 80%, at least 85%, at least 90%, at least 91%, at least 92%, at least 93%, at least 94%, at least 95%, at least 96%, at least 97%, at least 98%, or at least 99% sequence identity to any one of HC in (1)-(11); and/or, the LC has at least 70%, at least 80%, at least 85%, at least 90%, at least 91%, at least 92%, at least 93%, at least 94%, at least 95%, at least 96%, at least 97%, at least 98%, or at least 99% sequence identity to any one of LC in (1)-(11).

8. The human LIFR antigen binding protein according to any one of claims 1-7, wherein the human LIFR antigen binding protein comprises a chimeric antibody, a humanized antibody, or a fully human antibody.

9. The human LIFR antigen binding protein according to any one of claims 1-8, wherein the human LIFR antigen binding protein comprises a full-length antibody, Fab, Fab', F(ab')2, Fv, ScFv, di-scFv, a bispecific antibody, a multispecific antibody, a heavy chain antibody and/or a single domain antibody, or a monoclonal antibody and/or a polyclonal antibody derived from the above antibodies.

10. A nucleic acid molecule encoding the human LIFR antigen binding protein of any one of claims 1-9.

11. A vector comprising the nucleic acid molecule of claim 10.

12. A host cell comprising the nucleic acid molecule of claim 10 or the vector of claim 11.

13. A chimeric antigen receptor comprising the human LIFR antigen binding protein of any one of claims 1-9.

14. An immune cell comprising the chimeric antigen receptor of claim 13.

15. An antigen binding protein derivative comprising the human LIFR antigen binding protein of any one of claims 1-9 and a detectable label molecule, the detectable label molecule is an enzyme, a radionuclide, a fluorescent dye, a luminescent substance, or biotin.

16. A multispecific antibody comprising the human LIFR antigen binding protein of any one of claims 1-9, and another antibody or fragment thereof or antibody analog; wherein the multispecific antibody is a bispecific antibody or a tri-specific antibody or a tetra-specific antibody.

17. An antibody-drug conjugate comprising an antibody portion and a conjugated moiety, wherein the antibody portion comprises the human LIFR antigen binding protein of any one of claims 1-9, and the conjugated moiety includes, but is not limited to, a detectable label, a drug, a toxin, a cytokine, a radionuclide, an enzyme, or a combination thereof, and the antibody portion and the conjugated moiety are coupled through a chemical bond or a linker.

18. A pharmaceutical composition comprising the human LIFR antigen binding protein of any one of claims 1-9, the nucleic acid molecule of claim 10, and the vector of claim 11, the host cell of claim 12, the immune cell of claim 14, the antigen binding protein derivative of claim 15, the multispecific antibody of claim 16, and/or the antibody-drug conjugate of claim 17 and optionally a pharmaceutically acceptable carrier.

19. The pharmaceutical composition according to claim 18, wherein the pharmaceutical composition further comprises a therapeutic agent for combinational use, wherein the therapeutic agent includes, but is not limited to, a chemotherapeutic agent, a radiotherapeutic, an immunosuppressive agent, and a cellular toxic drug.

20. The method for producing a human LIFR antigen binding protein according to any one of claims 1-9, comprising cultivating the host cell of claim 12 under the condition that the antigen binding protein of any one of claims 1-9 can be expressed.

21. The method of claim 20, comprising the following steps:
(1) using LIFR-ECD protein as antigen to immunize mice;
(2) screening of antigen-specific B cells that secrete antibodies;
(3) restoring the heavy chain and light chain sequences of the antibody from the single B cell selected in step (2);
(4) introducing the antibody heavy chain and light chain recovered in step (3) into the host cell, and prepare the human LIFR antigen binding protein through cell culture and purification.

22. Use of the human LIFR antigen-binding protein of any one of claims 1-9, the nucleic acid molecule of claim 10, the vector of claim 11, the host cell of claim 12, the chimeric antigen receptor of claim 13, the immune cell according to claim 14, the antigen binding protein derivative of claim 15, the multispecific antibody of claim 16, the antibody-drug conjugate according to claim 17, and/or the pharmaceutical composition of claims 18-19 in the preparation of a LIF and/or LIFR blocking drug, a kit and/or a medical device.

23. Use of the human LIFR antigen binding protein of any one of claims 1-9, the nucleic acid molecule of claim 11, the vector of claim 12, the host cell of claim 13, the chimeric antigen receptor of claim 14, the immune cell of claim 15, the antigen binding protein derivative of claim 16, the multispecific antibody of claim 17, the antibody-drug conjugate of claim 18 and/or the pharmaceutical composition of claims 19-20 in the manufacture of a medicament, a kit and/or an administration device for preventing and/or treating a LIFR-positive disease.

24. The use of the human LIFR antigen binding protein of any one of claims 1-9, the antigen binding protein derivative of claim 15 in the preparation of a LIFR detection reagent or a kit.

25. A method for detecting LIFR using the human LIFR antigen binding protein of any one of claims 1-9 for qualitative or quantitative analysis to detect LIFR; preferably, the detection method is used for non-disease diagnosis or treatment purposes.

26. A method for the treatment of a LIFR-positive related disease, comprising administering an effective amount of the human LIFR antigen binding protein of any one of claims 1-9, the immune cell of claim 14, the antigen-binding protein derivative of claim 15, the multispecific antibody of claim 16, the antibody-drug conjugate of claim 17 and/or the pharmaceutical composition of claim 18-19 to a subject in need.

27. The use of claim 23 or the method of claim 26, wherein the LIFR-positive disease is a tumor, and the tumor includes but not limited to cholangiocarcinoma, colorectal cancer, glioma, prostate cancer, ovarian cancer, stomach cancer, nasopharyngeal cancer, breast cancer, bladder cancer, pancreatic cancer, and non-small cell lung cancer.

28. A kit comprising the human LIFR antigen binding protein of any one of claims 1-9, the nucleic acid molecule of claim 10, the vector of claim 11, the host cell of claim 12, the chimeric antigen receptor of claim 13, the immune cell of claim 14, the antigen binding protein derivative of claim 15, the multispecific antibody of claim 16, the antibody-drug conjugate of claim 17 and/or the pharmaceutical composition of claims 18-19, and optionally, instructions.

29. An administration device, comprising: (1) an infusion module for administering the pharmaceutical composition of claim 18 or 19 to a subject in need, and (2) a drug efficacy monitoring module optionally.

30. The use of LIFR antigen binding proteins in the manufacture of drugs for cancer treatment, wherein the cancer is selected from cholangiocarcinoma, colorectal cancer, glioma, prostate cancer, ovarian cancer, stomach cancer, nasopharyngeal cancer, breast cancer, bladder cancer, pancreatic cancer and non-small cell lung cancer.

31. The use according to claim 30, wherein the LIFR antigen binding protein is as described as any one of claims 1-9.
